# EUROPEAN PATENT SPECIFICATION

(11) **EP 3 320 857 B1**
(45) Date of publication and mention of the grant of the patent: **13.12.2023**
(21) Application number: 17201431.8
(22) Date of filing: 13.11.2017
(51) Int. Cl.: A61B 17/115, A61B 17/00, A61B 17/072, A61B 17/11

(54) **STAPLE FORMING POCKET CONFIGURATIONS FOR CIRCULAR SURGICAL STAPLER ANVIL**
KLAMMERFORMTASCHENKONFIGURATIONEN FÜR KREISFÖRMIGEN CHIRURGISCHEN KLAMMERAMBOSS
CONFIGURATIONS DE POCHE DE FORMATION D'AGRAFES POUR ENCLUME D'AGRAFEUSE CHIRURGICALE CIRCULAIRE

(30) Priority: 14.11.2016 US 201615350621
(43) Date of publication of application: 16.05.2018
(73) Proprietor: Ethicon LLC, 00969 Guaynabo (PR)
(72) Inventor: MILLER, Christopher C., Cincinnati, Ohio 45242 (US); SHELTON, IV, Frederick E., Cincinnati, Ohio 45242 (US)
(74) Representative: Carpmaels & Ransford LLP

(56) References cited:
- US-A1- 2014 151 430

## Description

### BACKGROUND

In some surgical procedures (e.g., colorectal, bariatric, thoracic, etc.), portions of a patient's digestive tract (e.g., the gastrointestinal tract and/or esophagus, etc.) may be cut and removed to eliminate undesirable tissue or for other reasons. Once the tissue is removed, the remaining portions of the digestive tract may be coupled together in an end-to-end anastomosis. The end-to-end anastomosis may provide a substantially unobstructed flow path from one portion of the digestive tract to the other portion of the digestive tract, without also providing any kind of leaking at the site of the anastomosis.

One example of an instrument that may be used to provide an end-to-end anastomosis is a circular stapler. Some such staplers are operable to clamp down on layers of tissue, cut through the clamped layers of tissue, and drive staples through the clamped layers of tissue to substantially seal the layers of tissue together near the severed ends of the tissue layers, thereby joining the two severed ends of the anatomical lumen together. The circular stapler may be configured to sever the tissue and seal the tissue substantially simultaneously. For instance, the circular stapler may sever excess tissue that is interior to an annular array of staples at an anastomosis, to provide a substantially smooth transition between the anatomical lumen sections that are joined at the anastomosis. Circular staplers may be used in open procedures or in endoscopic procedures. In some instances, a portion of the circular stapler is inserted through a patient's naturally occurring orifice.

Examples of circular staplers are described in U.S. Pat. No. 5,205,459, entitled "Surgical Anastomosis Stapling Instrument," issued April 27, 1993; U.S. Pat. No. 5,271,544, entitled "Surgical Anastomosis Stapling Instrument," issued December 21, 1993; U.S. Pat. No. 5,275,322, entitled "Surgical Anastomosis Stapling Instrument," issued January 4, 1994; U.S. Pat. No. 5,285,945, entitled "Surgical Anastomosis Stapling Instrument," issued February 15, 1994; U.S. Pat. No. 5,292,053, entitled "Surgical Anastomosis Stapling Instrument," issued March 8, 1994; U.S. Pat. No. 5,333,773, entitled "Surgical Anastomosis Stapling Instrument," issued August 2, 1994; U.S. Pat. No. 5,350,104, entitled "Surgical Anastomosis Stapling Instrument," issued September 27, 1994; and U.S. Pat. No. 5,533,661, entitled "Surgical Anastomosis Stapling Instrument," issued July 9, 1996; and U.S. Pat. No. 8,910,847, entitled "Low Cost Anvil Assembly for a Circular Stapler," issued December 16, 2014.

Some circular staplers may include a motorized actuation mechanism. Examples of circular staplers with motorized actuation mechanisms are described in U.S. Pub. No. 2015/0083772, entitled "Surgical Stapler with Rotary Cam Drive and Return," published March 26, 2015; U.S. Pub. No. 2015/0083773, entitled "Surgical Stapling Instrument with Drive Assembly Having Toggle Features," published March 26, 2015; U.S. Pub. No. 2015/0083774, entitled "Control Features for Motorized Surgical Stapling Instrument," published March 26, 2015; and U.S. Pub. No. 2015/0083775, entitled "Surgical Stapler with Rotary Cam Drive," published March 26, 2015.

While various kinds of surgical stapling instruments and associated components have been made and used, it is believed that no one prior to the inventor(s) has made or used the invention described in the appended claims.

### SUMMARY OF THE INVENTION

The present invention provides an apparatus as recited in claim 1. Optional features are recited in the dependent claims.

### BRIEF DESCRIPTION OF THE DRAWINGS

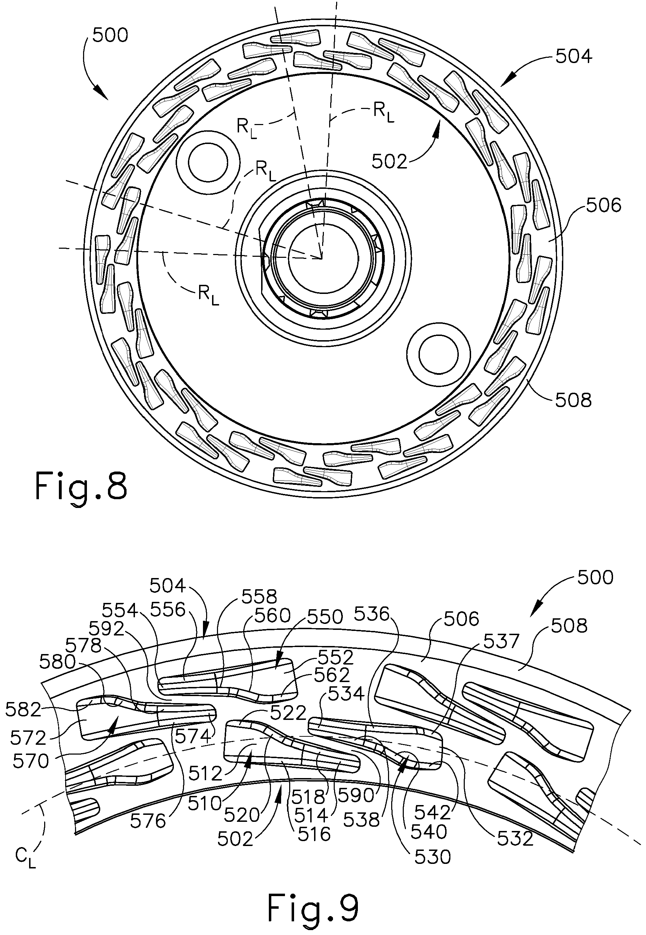

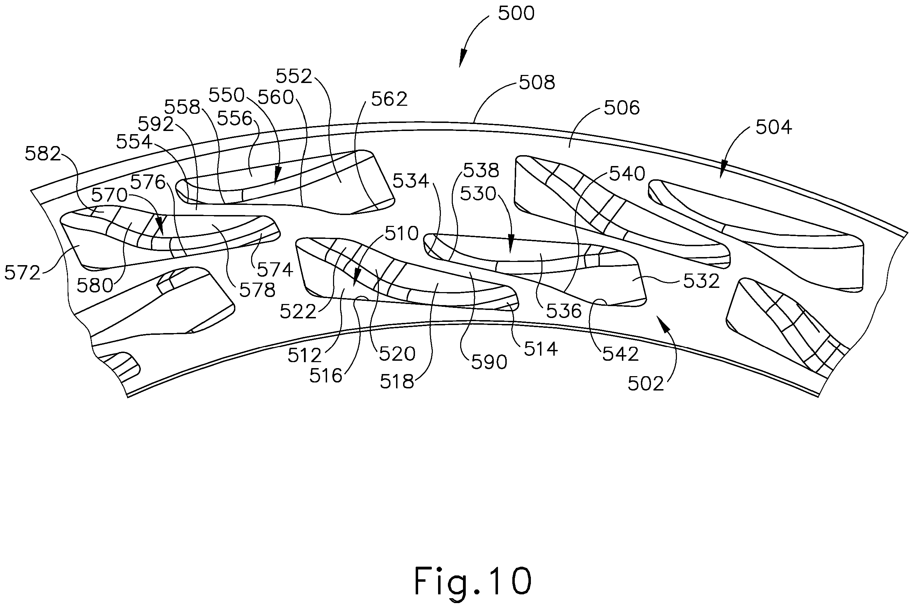

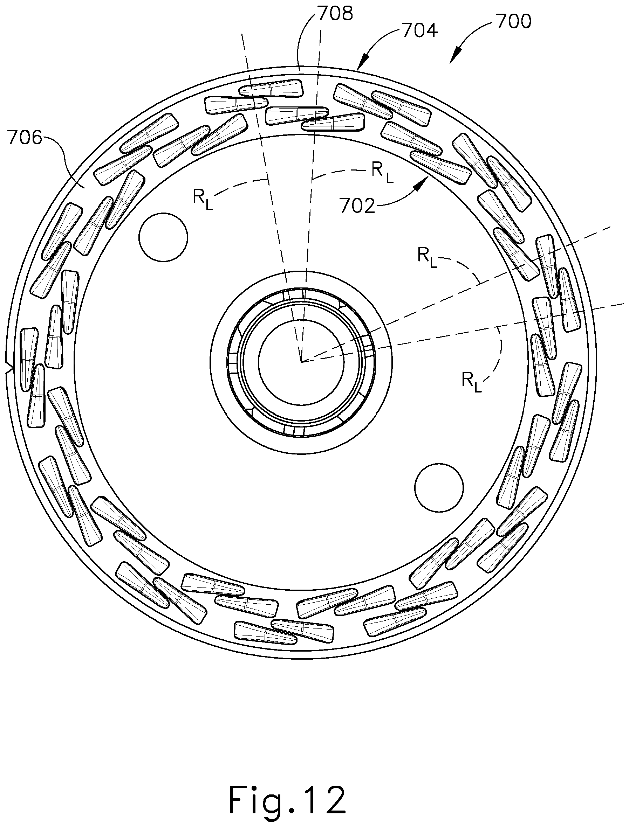

While the specification concludes with claims which particularly point out and distinctly claim this technology, it is believed this technology will be better understood from the following description of certain staplers, stapler head assemblies and anvils taken in conjunction with the accompanying drawings, in which like reference numerals identify the same elements. Figures 12 to 15, 18 and 22 show features of apparatuses that fall within the scope of the claims. Figures 8 to 11 show anvils that do not fall within the scope of the claims. All other examples are useful for highlighting features of staplers and stapler head assemblies with which the claimed apparatus can be used. In the drawings:
FIG. 1 depicts a perspective view of an exemplary circular stapler;
FIG. 2 depicts a perspective view of the circular stapler of FIG. 1, with a battery pack removed from a handle assembly and an anvil removed from a stapling head assembly;
FIG. 3 depicts a perspective view of the anvil of the circular stapler of FIG. 1;
FIG. 4 depicts a perspective view of the stapling head assembly of the circular stapler of FIG. 1;
FIG. 5 depicts an exploded perspective view of the stapling head assembly of FIG. 4;
FIG. 6 depicts an exploded perspective view of the circular stapler of FIG. 1, with portions of the shaft assembly shown separately from each other;
FIG. 7A depicts a cross-sectional side view of the anvil of FIG. 3 positioned within a first section of a digestive tract and the stapling head assembly of FIG. 4 positioned in a second section of the digestive tract, with the anvil separated from the stapling head assembly;
FIG. 7B depicts a cross-sectional side view of the anvil of FIG. 3 positioned within the first section of the digestive tract and the stapling head assembly of FIG. 4 positioned in the second section of the digestive tract, with the anvil secured to the stapling head assembly;
FIG. 7C depicts a cross-sectional side view of the anvil of FIG. 3 positioned within the first section of the digestive tract and the stapling head assembly of FIG. 4 positioned in the second section of the digestive tract, with the anvil retracted toward the stapling head assembly to thereby clamp tissue between the anvil and the stapling head assembly;
FIG. 7D depicts a cross-sectional side view of the anvil of FIG. 3 positioned within the first section of the digestive tract and the stapling head assembly of FIG. 4 positioned in the second section of the digestive tract, with the stapling head assembly actuated to sever and staple the clamped tissue;
FIG. 7E depicts a cross-sectional side view of the first and second sections of the digestive tract of FIG. 7A joined together at an end-to-end anastomosis;
FIG. 8 depicts a bottom plan view of an exemplary alternative anvil not according to the invention that may be used with the circular stapler of FIG. 1;
FIG. 9 depicts an enlarged bottom plan view of a portion of the anvil of FIG. 8;
FIG. 10 depicts an enlarged perspective view of a portion of the anvil of FIG. 8;
FIG. 11 depicts an enlarged bottom plan view of a portion of an exemplary alternative anvil not according to the invention that may be used with the circular stapler of FIG. 1;
FIG. 12 depicts a bottom plan view of another exemplary alternative anvil that may be used with the circular stapler of FIG. 1;
FIG. 13 depicts an enlarged bottom plan view of a portion of the anvil of FIG. 12;
FIG. 14 depicts an enlarged perspective view of a portion of the anvil of FIG. 12;
FIG. 15 depicts an enlarged bottom plan view of another exemplary alternative anvil that may be used with the circular stapler of FIG. 1;
FIG. 16 depicts an enlarged top plan view of a portion of an array of staples formed using the anvil of FIG. 15;
FIG. 17 depicts an enlarged perspective view of a portion of an array of staples formed using the anvil of FIG. 15;
FIG. 18 depicts an enlarged bottom plan view of another exemplary alternative anvil that may be used with the circular stapler of FIG. 1;
FIG. 19 depicts an enlarged top plan view of a portion of an array of staples formed using the anvil of FIG. 18;
FIG. 20 depicts an enlarged perspective view of a portion of an array of staples formed using the anvil of FIG. 18;
FIG. 21 depicts a top plan view of a portion of an exemplary alternative stapling head assembly that may be incorporated into the circular stapler of FIG. 1; and
FIG. 22 depicts a bottom plan view of a portion of an exemplary alternative anvil that may be used in conjunction with the stapling head assembly of FIG. 21.

The drawings are not intended to be limiting in any way, and it is contemplated that the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, and advantages of the technology will become apparent to those skilled in the art from the following description. The drawings and descriptions should be regarded as illustrative in nature and not restrictive.

### I. Overview of Exemplary Circular Stapling Surgical Instrument

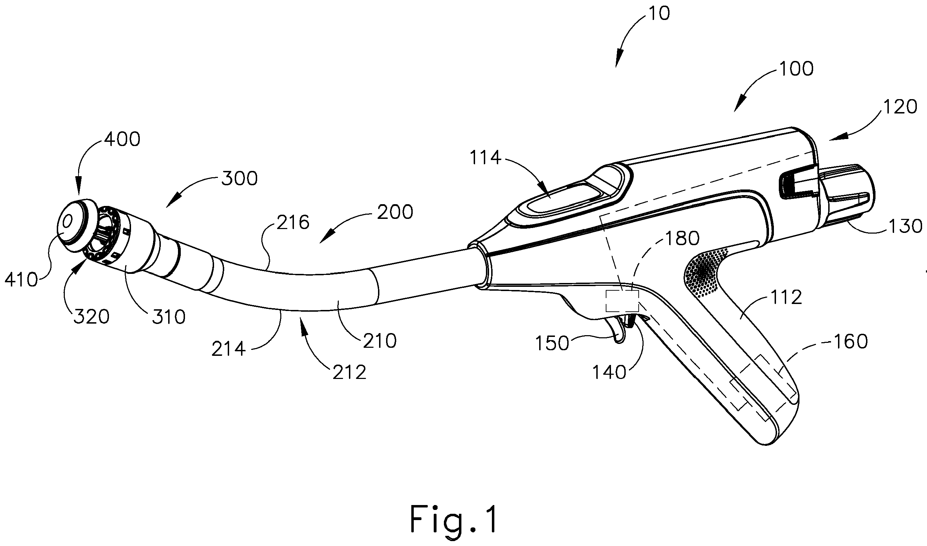

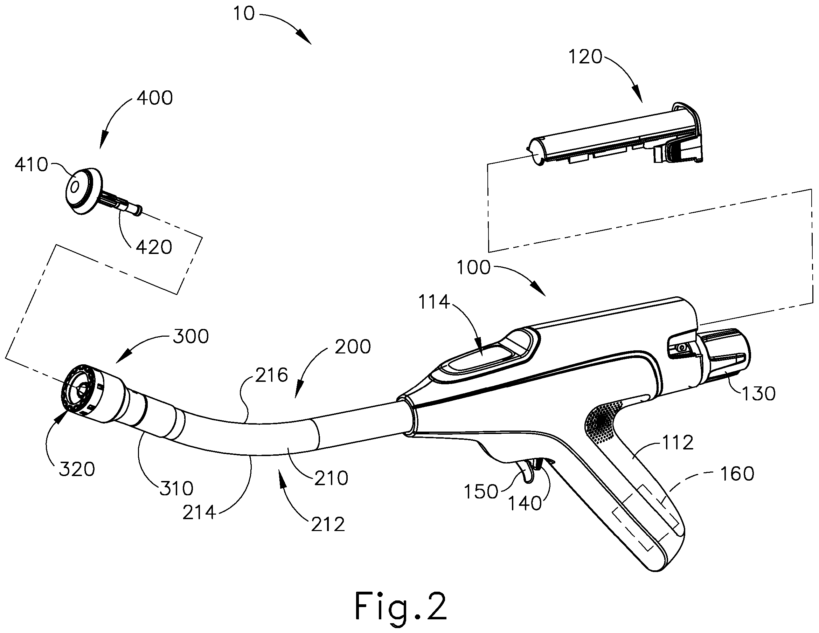

FIGS. 1-2 depict an exemplary surgical circular stapling instrument (10) that may be used to provide an end-to-end anastomosis between two sections of an anatomical lumen such as a portion of a patient's digestive tract. Instrument (10) of this example comprises a handle assembly (100), a shaft assembly (200), a stapling head assembly (300), an anvil (400), and a removable battery pack (120). Each of these components will be described in greater detail below. It should be understood that, in addition to or in lieu of the following, instrument (10) may be further constructed and operable in accordance with at least some of the teachings of U.S. Pat. App. No. 14/751,612, entitled "Method of Applying an Annular Array of Staples to Tissue," filed June 26, 2015; U.S. Pat. No. 5,205,459; U.S. Pat. No. 5,271,544; U.S. Pat. No. 5,275,322; U.S. Pat. No. 5,285,945; U.S. Pat. No. 5,292,053; U.S. Pat. No. 5,333,773; U.S. Pat. No. 5,350,104; U.S. Pat. No. 5,533,661; and/or U.S. Pat. No. 8,910,847. Still other suitable configurations will be apparent to one of ordinary skill in the art in view of the teachings herein.

### A. Exemplary Tissue Engagement Features of Circular Stapling Instrument

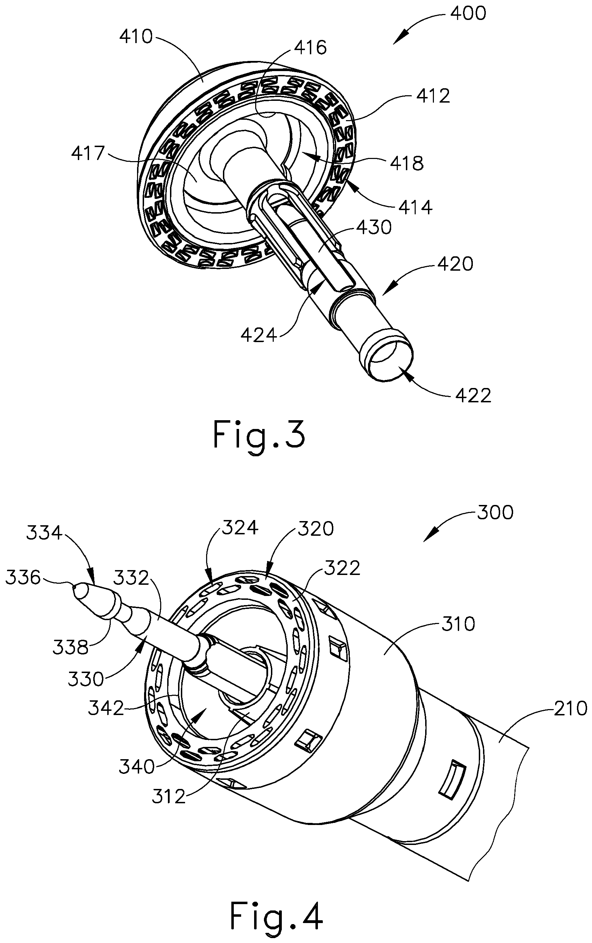

As best seen in FIG. 3, anvil (400) of the present example comprises a head (410) and a shank (420). Head (410) includes a proximal surface (412) that defines a plurality of staple forming pockets (414). Staple forming pockets (414) are arranged in two concentric annular arrays in the present example. Staple forming pockets (414) are configured to deform staples as the staples are driven into staple forming pockets (414) (e.g., deforming a generally "U" shaped staple into a "B" shape as is known in the art). Shank (420) defines a bore or lumen (422) and includes a pair of pivoting latch members (430) positioned in bore (422). Each latch member (430) includes features that allows anvil (400) to be removably secured to a trocar (330) of stapling head assembly (300) as will be described in greater detail below. It should be understood, however, that anvil (400) may be removably secured to a trocar (330) using any other suitable components, features, or techniques.

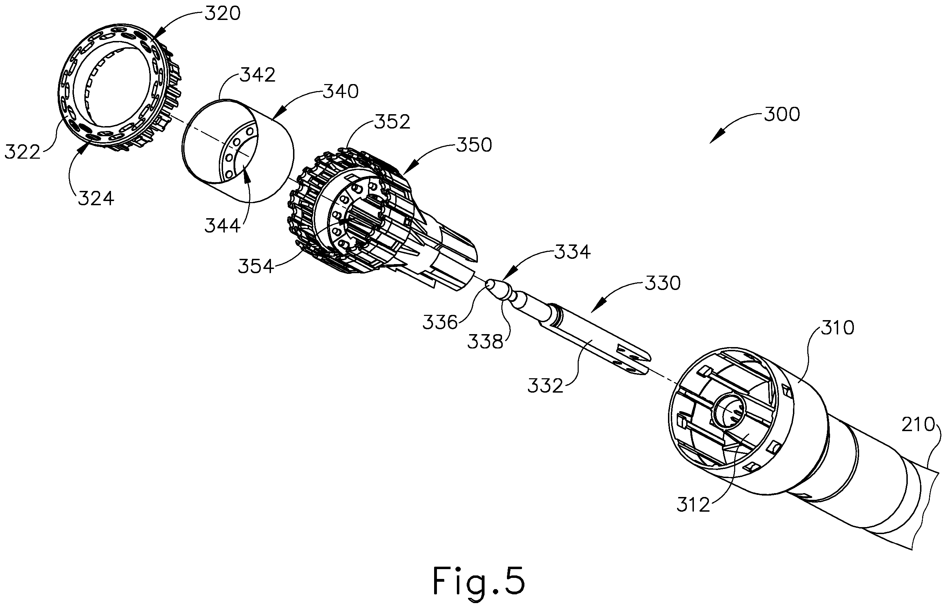

Stapling head assembly (300) is located at the distal end of shaft assembly (200). As shown in FIGS. 1-2, anvil (400) is configured to removably couple with shaft assembly (200), adjacent to stapling head assembly (300). As will be described in greater detail below, anvil (400) and stapling head assembly (300) are configured to cooperate to manipulate tissue in three ways, including clamping the tissue, cutting the tissue, and stapling the tissue. As best seen in FIGS. 4-5, stapling head assembly (300) of the present example comprises a tubular casing (310) housing a slidable staple driver member (350). A cylindraceous inner core member (312) extends distally within tubular casing (310). Tubular casing (310) is fixedly secured to an outer sheath (210) of shaft assembly (200), such that tubular casing (310) serves as a mechanical ground for stapling head assembly (300).

Trocar (330) is positioned coaxially within inner core member (312) of tubular casing (310). Trocar (330) is operable to translate distally and proximally relative to tubular casing (310) in response to rotation of a knob (130) located at the proximal end of handle assembly (100). Trocar (330) comprises a shaft (332) and ahead (334). Head (334) includes a pointed tip (336) and an inwardly extending proximal surface (338). Head (334) and the distal portion of shaft (332) are configured for insertion in bore (422) of anvil (420). Proximal surface (338) is configured to complement features of latch members (430) to provide a snap fit between anvil (400) and trocar (330).

Staple driver member (350) is operable to actuate longitudinally within tubular casing (310) in response to activation of motor (160) as will be described in greater detail below. Staple driver member (350) includes two distally presented concentric annular arrays of staple drivers (352). Staple drivers (352) are arranged to correspond with the arrangement of staple forming pockets (414) described above. Thus, each staple driver (352) is configured to drive a corresponding staple into a corresponding staple forming pocket (414) when stapling head assembly (300) is actuated. Staple driver member (350) also defines a bore (354) that is configured to coaxially receive core member (312) of tubular casing (310).

A cylindraceous knife member (340) is coaxially positioned within staple driver member (350). Knife member (340) includes a distally presented, sharp circular cutting edge (342). Knife member (340) is sized such that knife member (340) defines an outer diameter that is smaller than the diameter defined by the inner annular array of staple drivers (352). Knife member (340) also defines an opening that is configured to coaxially receive core member (312) of tubular casing (310).

A deck member (320) is fixedly secured to tubular casing (310). Deck member (320) includes a distally presented deck surface (322) defining two concentric annular arrays of staple openings (324). Staple openings (324) are arranged to correspond with the arrangement of staple drivers (352) and staple forming pockets (414) described above. Thus, each staple opening (324) is configured to provide a path for a corresponding staple driver (352) to drive a corresponding staple through deck member (320) and into a corresponding staple forming pocket (414) when stapling head assembly (300) is actuated. It should be understood that the arrangement of staple openings (322) may be modified just like the arrangement of staple forming pockets (414) as described above. It should also be understood that various structures and techniques may be used to contain staples within stapling head assembly (300) before stapling head assembly (300) is actuated. Deck member (320) defines an inner diameter that is just slightly larger than the outer diameter defined by knife member (340). Deck member (320) is thus configured to allow knife member (340) to translate distally to a point where cutting edge (342) is distal to deck surface (322).

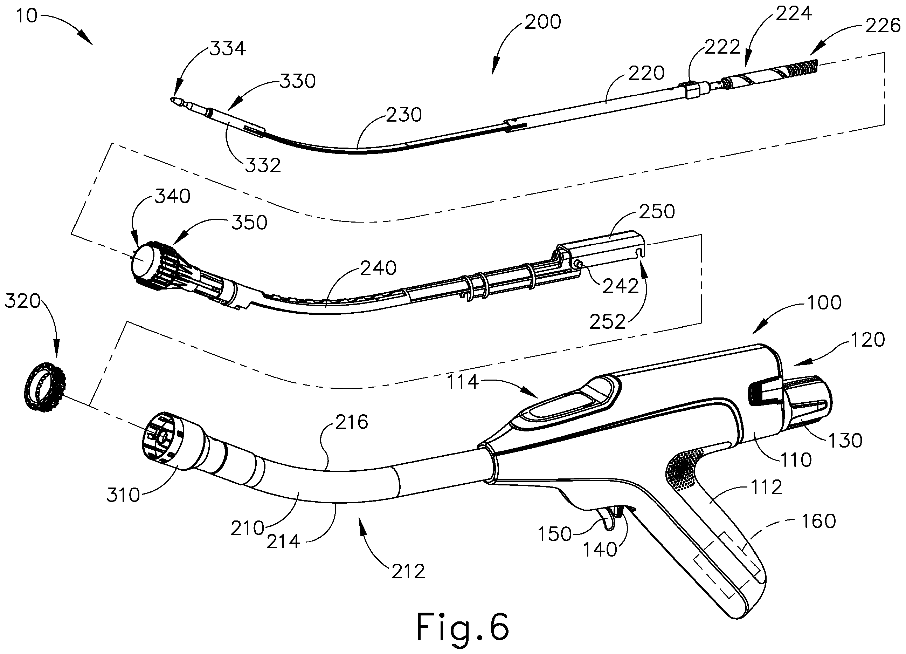

FIG. 6 shows various components of shaft assembly (200), which extends distally from handle assembly (100) and couples components of stapling head assembly (300) with components of handle assembly (100). In particular, and as noted above, shaft assembly (200) includes an outer sheath (210) that extends between handle assembly (100) and tubular casing (310). In the present example, outer sheath (210) is rigid and includes a preformed curved section (212) that is configured to facilitate positioning of stapling head assembly (300) within a patient's colon as described below. Curved section (212) includes an inner curve (216) and an outer curve (214).

Shaft assembly (200) further includes a trocar actuation rod (220) and a trocar actuation band assembly (230). The distal end of trocar actuation band assembly (230) is fixedly secured to the proximal end of trocar shaft (332). The proximal end of trocar actuation band assembly (230) is fixedly secured to the distal end of trocar actuation rod (220), such that trocar (330) will translate longitudinally relative to outer sheath (210) in response to translation of trocar actuation band assembly (230) and trocar actuation rod (220) relative to outer sheath (210). Trocar actuation band assembly (230) is configured to flex such that trocar actuation band assembly (230) may follow along the preformed curve in shaft assembly (200) as trocar actuation band assembly (230) is translated longitudinally relative to outer sheath (210). However, trocar actuation band assembly (230) has sufficient column strength and tensile strength to transfer distal and proximal forces from trocar actuation rod (220) to trocar shaft (332). Trocar actuation rod (220) is rigid. A clip (222) is fixedly secured to trocar actuation rod (220) and is configured to cooperate with complementary features within handle assembly (100) to prevent trocar actuation rod (220) from rotating within handle assembly (100) while still permitting trocar actuation rod (220) to translate longitudinally within handle assembly (100). Trocar actuation rod (220) further includes a coarse helical threading (224) and a fine helical threading (226).

Shaft assembly (200) further includes a stapling head assembly driver (240) that is slidably received within outer sheath (210). The distal end of stapling head assembly driver (240) is fixedly secured to the proximal end of staple driver member (350). The proximal end of stapling head assembly driver (240) is secured to a drive bracket (250) via a pin (242). It should therefore be understood that staple driver member (350) will translate longitudinally relative to outer sheath (210) in response to translation of stapling head assembly driver (240) and drive bracket (250) relative to outer sheath (210). Stapling head assembly driver (240) is configured to flex such that stapling head assembly driver (240) may follow along the preformed curve in shaft assembly (200) as stapling head assembly driver (240) is translated longitudinally relative to outer sheath (210). However, stapling head assembly driver (240) has sufficient column strength to transfer distal forces from drive bracket (250) to staple driver member (350).

### B. Exemplary User Input Features of Circular Stapling Instrument

As shown in FIG. 1, handle assembly (100) includes a pistol grip (112) and several components that are operable to actuate anvil (400) and stapling head assembly (300). In particular, handle assembly (100) includes knob (130), a safety trigger (140) a firing trigger (150), a motor (160), and a motor activation module (180). Knob (130) is coupled with trocar actuation rod (220) via a nut (not shown), such that coarse helical threading (224) will selectively engage a thread engagement feature within the interior of the nut; and such that fine helical threading (226) will selectively engage a thread engagement feature within the interior of knob (130). These complementary structures are configured such that trocar actuation rod (220) will first translate proximally at a relatively slow rate, then translate proximally at a relatively fast rate, in response to rotation of knob (130).

It should be understood that when anvil (400) is coupled with trocar (330), rotation of knob (130) will provide corresponding translation of anvil relative to stapling head assembly (300). It should also be understood that knob (130) may be rotated in a first angular direction (e.g., clockwise) to retract anvil (400) toward stapling head assembly (300); and in a second angular direction (e.g., counterclockwise) to advance anvil (500) away from stapling head assembly (300). Knob (130) may thus be used to adjust the gap distance between opposing surfaces (412, 322) of anvil (400) and stapling head assembly (300) until a suitable gap distance has been achieved.

In the present example, handle assembly (100) comprises a user feedback feature (114) that is configured to provide the operator with visual feedback indicating the positioning of anvil (400) in relation to stapling assembly (300). The operator may thus observe user feedback feature (114) while rotating knob (130), to confirm whether the suitable gap distance between anvil (400) and stapling assembly (300) has been achieved. By way of example only, user feedback feature (114) may be configured and operable in accordance with at least some of the teachings of U.S. Pat. App. No. 14/751,612, entitled "Method of Applying an Annular Array of Staples to Tissue," filed June 26, 2015. Other suitable forms of providing user feedback will be apparent to those of ordinary skill in the art in view of the teachings herein.

Firing trigger (150) is operable to activate motor (160) to thereby actuate stapling head assembly (300). Safety trigger (140) is operable to selectively block actuation of firing trigger (150) based on the longitudinal position of anvil (400) in relation to stapling head assembly (300). Handle assembly (100) also includes components that are operable to selectively lock out both triggers (140, 150) based on the position of anvil (400) relative to stapling head assembly (300). When triggers (140, 150) are locked out, firing trigger (150) is prevented from initiating actuation of stapling head assembly (300). Thus, trigger (150) is only operable to initiate actuation of stapling head assembly (300) when the position of anvil (400) relative to stapling head assembly (300) is within a predefined range.

In the present example, firing trigger (150) of the present example includes an integral actuation paddle, such as the paddle shown and described in U.S. Patent App. No. 14/751,231, entitled "Surgical Stapler with Reversible Motor," filed June 26, 2015. The paddle is configured to actuate a switch of motor activation module (180) (FIG. 1) when firing trigger (150) is pivoted to a fired position. Motor activation module (180) is in communication with battery pack (120) and motor (160), such that motor activation module (180) is configured to provide activation of motor (160) with electrical power from battery pack (120) in response to the paddle actuating the switch of motor activation module (180). Thus, motor (160) will be activated when firing trigger (150) is pivoted. This activation of motor (160) will actuate stapling head assembly (300) as described in greater detail below.

Battery pack (120) is operable to provide electrical power to a motor (160) as noted above. Battery pack (120) may be removably coupled with handle assembly (100) through a snap fit or in any other suitable fashion. It should be understood that battery pack (120) and handle assembly (100) may have complementary electrical contacts, pins and sockets, and/or other features that provide paths for electrical communication from battery pack (120) to electrically powered components in handle assembly (100) when battery pack (120) is coupled with handle assembly (100). It should also be understood that, in some versions, battery pack (120) is unitarily incorporated within handle assembly (100) such that battery back (120) cannot be removed from handle assembly (100).

### C. Exemplary Anastomosis Procedure with Circular Stapling Instrument

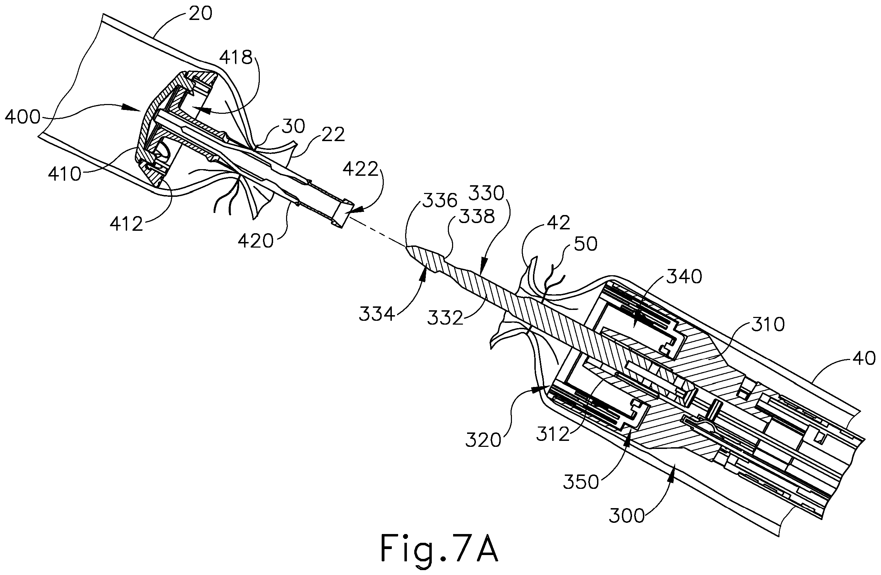

FIGS. 7A-7E show instrument (10) being used to form an anastomosis (70) between two tubular anatomical structures (20, 40). By way of example only, the tubular anatomical structures (20, 40) may comprise sections of a patient's esophagus, sections of a patient's colon, other sections of the patient's digestive tract, or any other tubular anatomical structures. In some versions, one or more diseased portions of a patient's colon are removed, with the tubular anatomical structures (20, 40) of FIGS. 7A-7E representing the remaining severed portions of the colon.

As shown in FIG. 7A, anvil (400) is positioned in one tubular anatomical structure (20) and stapling head assembly (300) is positioned in another tubular anatomical structure (40). In versions where tubular anatomical structures (20, 40) comprise sections of a patient's colon, stapling head assembly (300) may be inserted via the patient's rectum. It should also be understood that the procedure depicted in FIGS. 7A-7E is an open surgical procedure, though the procedure may instead be performed laparoscopically. By way of example only, the surgical procedure may be performed laparoscopically in accordance with at least some of the teachings of U.S. Pub. No. 2016/0100837, entitled "Staple Cartridge," published April 14, 2016; and/or U.S. Pat. App. No. 14/864,310, entitled "Apparatus and Method for Forming a Staple Line with Trocar Passageway," filed September 24, 2015. Various other suitable ways in which instrument (10) may be used to form an anastomosis (70) in a laparoscopic procedure will be apparent to those of ordinary skill in the art in view of the teachings herein.

As shown in FIG. 7A, anvil (400) is positioned in tubular anatomical structure (20) such that shank (420) protrudes from the open severed end (22) of tubular anatomical structure (20). A purse-string suture (30) is provided about a mid-region of shank (420) to generally secure the position of anvil (400) in tubular anatomical structure (20). Similarly, stapling head assembly (300) is positioned in tubular anatomical structure (40) such that trocar (330) protrudes from the open severed end (42) of tubular anatomical structure (20). A purse-string suture (50) is provided about a mid-region of shaft (332) to generally secure the position of stapling head assembly (300) in tubular anatomical structure (40).

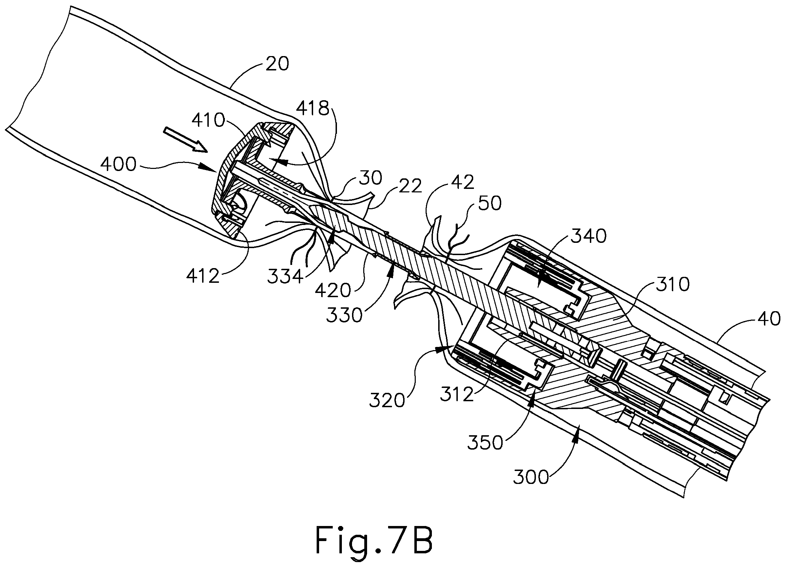

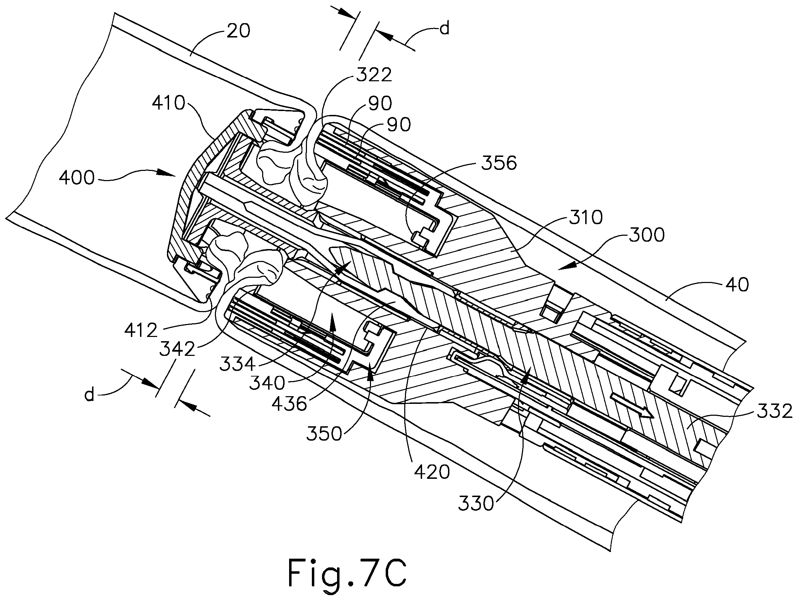

Next, anvil (400) is secured to trocar (330) by inserting trocar (330) into bore (422) as shown in FIG. 7B. Latch members (430) engage head (334) of trocar (330), thereby providing a secure fit between anvil (400) and trocar (330). The operator then rotates knob (130) while holding handle assembly (100) stationary via pistol grip (112). This rotation of knob (130) causes trocar (330) and anvil (400) to retract proximally, as described above. As shown in FIG. 7C, this proximal retraction of trocar (330) and anvil (400) compresses the tissue of tubular anatomical structures (20, 40) between surfaces (412, 322) of anvil (400) and stapling head assembly (300). The operator observes user feedback feature (114) to determine whether the gap distance (d) between opposing surfaces (412, 322) of anvil (400) and stapling head assembly (300) is appropriate; and makes any necessary adjustments via knob (130).

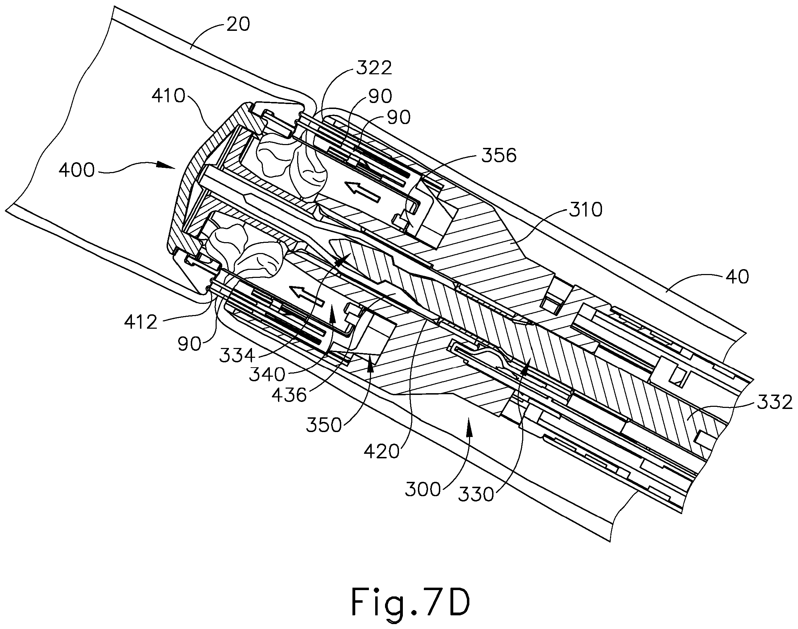

Once the operator has appropriately set the gap distance (d) via knob (130), the operator actuates safety trigger (140) to enable actuation of firing trigger (150). The operator then actuates firing trigger (150). This actuation of firing trigger (150) in turn actuates a switch of motor activation module (180), which in turn activates motor (160) to thereby actuate stapling head assembly (300) by driving knife member (340) and staple driver member (350) distally as shown in FIG. 7D. As knife member (340) translates distally, cutting edge (342) of knife member (340) cooperates with inner edge (416) of anvil (400), thereby shearing excess tissue that is positioned within annular recess (418) of anvil (400) and the interior of knife member (340).

As shown in FIG. 4, anvil (400) of the present example includes a breakable washer (417) within annular recess (418). This washer (417) is broken by knife member (340) when knife member (340) completes a full distal range of motion from the position shown in FIG. 7C to the position shown in FIG. 7D. The drive mechanism for knife member (340) may provide an increasing mechanical advantage as knife member (340) reaches the end of its distal movement, thereby providing greater force by which to break washer (417). Of course, breakable washer (417) may be omitted entirely in some versions. In versions where washer (417) is included, it should be understood that washer (417) may also serve as a cutting board for knife member (340) to assist in cutting of tissue. Such a cutting technique may be employed in addition to or in lieu of the above-noted shearing action between inner edge (416) and cutting edge (342).

As staple driver member (350) translates distally from the position shown in FIG. 7C to the position shown in FIG. 7D, staple driver member (350) drives staples (90) through the tissue of tubular anatomical structures (20, 40) and into staple forming pockets (414) of anvil (400). Staple forming pockets (414) deform the driven staples (90) into a "B" shape as is known in the art. The formed staples (90) thus secure the ends of tissue together, thereby coupling tubular anatomical structure (20) with tubular anatomical structure (40).

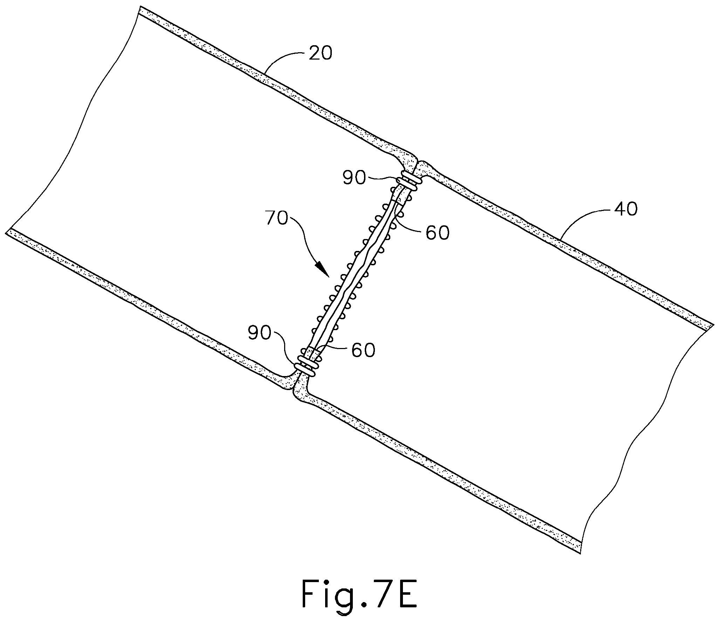

After the operator has actuated stapling head assembly (300) as shown in FIG. 7D, the operator rotates knob (130) to drive anvil (400) distally away from stapling head assembly (300), increasing the gap distance (d) to facilitate release of the tissue between surfaces (412, 322). The operator then removes instrument (10) from the patient, with anvil (400) still secured to trocar (330). Referring back to the example where the tubular anatomical structures (20, 40) comprise sections of a patient's colon, instrument (10) may be removed via the patient's rectum. With instrument (10) removed, the tubular anatomical structures (20, 40) are left secured together by two annular arrays of staples (90) at an anastomosis (70) as shown in FIG. 7E. The inner diameter of the anastomosis (70) is defined by the severed edge (60) left by knife member (340).

### II. Exemplary Alternative Anvils

In some instances, it may be desirable to change the configuration and arrangement of staple forming pockets (414) in anvil (400). It should be understood that reconfiguring and rearranging staple forming pockets (414) may result in reconfiguration and rearrangement of staples (90) that are formed by staple forming pockets (414). For instance, the configuration and arrangement of staple forming pockets (414) may affect the structural integrity of an anastomosis (70) that is secured by staples (90). In addition, the configuration and arrangement of staple forming pockets (414) may affect the hemostasis that is achieved at an anastomosis (70) that is secured by staples (90). The following description relates to several exemplary variations of anvil (400), providing staple forming pocket configurations and arrangements that differ from those of staple forming pockets (414).

It should be understood that the various alternatives to anvil (400) described below may be readily used with instrument (10), in place of anvil (400). It should also be understood that, in some instances, the configuration and arrangement of staple openings (324) in deck member (320) may need to be varied in order to complement the configuration and arrangement of the alternative staple forming pockets described below. Various suitable ways in which the alternatives to anvil (400) described below may be incorporated into instrument (10) will be apparent to those of ordinary skill in the art in view of the teachings herein.

### A. Exemplary Anvil with Symmetric Arrays of Staple Forming Pockets having Staple Leg Deflection Wall with Dogleg Configuration

FIGS. 8-10 show an exemplary alternative anvil (500) that may be used with a modified version of instrument (10). Anvil (500) of this example is configured and operable just like anvil (400), except for the differences described below. Anvil (500) of the present example comprises a proximal surface (506) that defines an inner annular array (502) of staple forming pockets (510, 530) and an outer annular array (504) of staple forming pockets (550, 570). A chamfered edge (508) extends about the outer perimeter of proximal surface (506). It should be understood that anvil (500) may be secured to trocar (330), that proximal surface (506) may be used to compress tissue against deck surface (322), and that staple driver (352) may drive staples (90) through tissue into staple forming pockets (510, 530, 550, 570) in order to thereby form staples (90) in the tissue.

As best seen in FIGS. 9-10, each staple forming pocket (510) comprises a staple entry surface (512) and a staple exit surface (514). Surfaces (512, 514) are contiguous with each other and define a concave recess. The concave recess formed by surfaces (512, 514) is further defined by an inner wall (516), a first outer wall (518), a second outer wall (520), and a third outer wall (522). In the present example, walls (516, 518, 520, 522) are each substantially flat. Wall (518) defines a relatively narrow, tapered gap with wall (516). Wall (522) defines a relatively wide gap with wall (516). Wall (520) is obliquely angled, providing an inwardly sloped transition from wall (522) to wall (518). Thus, walls (518, 520, 522) together provide a dogleg configuration. The edge connecting wall (516) with wall (522) is substantially straight in this example. Similarly, the edge connecting wall (516) with wall (518) is substantially straight in this example.

It should be understood that when a first leg of staple (90) is driven into staple forming pocket (510), the first leg first encounters staple entry surface (512), bends generally toward the second leg of staple (90) along a first plane that is orthogonal to the axis of the unformed first leg, and then bends proximally back generally toward the crown of staple (90). In addition, the first leg will eventually encounter wall (520), which will provide a cam surface bending the first leg along a second plane that is orthogonal to the axis of the unformed first leg. In particular, wall (520) and then wall (518) will deflect the first leg radially inwardly toward the central axis of anvil (500). Thus, staple forming pocket (510) will ultimately deflect a first leg of a staple (90) proximally and radially inwardly. Wall (516) will restrict the degree to which the first leg of staple (90) deflects radially inwardly.

Each staple forming pocket (530) comprises a staple entry surface (532) and a staple exit surface (534). Surfaces (532, 534) are contiguous with each other and define a concave recess. The concave recess formed by surfaces (532, 534) is further defined by an outer wall (536), a first inner wall (538), a second inner wall (540), and a third inner wall (542). In the present example, walls (536, 538, 540, 542) are each substantially flat. Wall (538) defines a relatively narrow, tapered gap with wall (536). Wall (542) defines a relatively wide gap with wall (536). Wall (540) is obliquely angled, providing an outwardly sloped transition from wall (542) to wall (538). Thus, walls (538, 540, 542) together provide a dogleg configuration. The edge connecting wall (536) with wall (542) is substantially straight in this example. Similarly, the edge connecting wall (536) with wall (538) is substantially straight in this example.

It should be understood that when a second leg of staple (90) is driven into staple forming pocket (530), the second leg first encounters staple entry surface (532), bends generally toward the first leg of staple (90) along a first plane that is orthogonal to the axis of the unformed second leg, and then bends proximally back generally toward the crown of staple (90). In addition, the second leg will eventually encounter wall (540), which will provide a cam surface bending the second leg along a second plane that is orthogonal to the axis of the unformed second leg. In particular, wall (540) and then wall (538) will deflect the second leg radially outwardly away from the central axis of anvil (500). Thus, staple forming pocket (530) will ultimately deflect a second leg of a staple (90) proximally and radially outwardly. Wall (536) will restrict the degree to which the second leg of staple (90) deflects radially outwardly.

Each staple forming pocket (550) comprises a staple entry surface (552) and a staple exit surface (554). Surfaces (552, 554) are contiguous with each other and define a concave recess. The concave recess formed by surfaces (552, 554) is further defined by an outer wall (556), a first inner wall (558), a second inner wall (560), and a third inner wall (562). In the present example, walls (556, 558, 560, 562) are each substantially flat. Wall (558) defines a relatively narrow, tapered gap with wall (556). Wall (562) defines a relatively wide gap with wall (556). Wall (560) is obliquely angled, providing an outwardly sloped transition from wall (562) to wall (558). Thus, walls (558, 560, 562) together provide a dogleg configuration. The edge connecting wall (556) with wall (562) is substantially straight in this example. Similarly, the edge connecting wall (556) with wall (558) is substantially straight in this example.

It should be understood that when a second leg of staple (90) is driven into staple forming pocket (550), the second leg first encounters staple entry surface (552), bends generally toward the first leg of staple (90) along a first plane that is orthogonal to the axis of the unformed second leg, and then bends proximally back generally toward the crown of staple (90). In addition, the second leg will eventually encounter wall (560), which will provide a cam surface bending the second leg along a second plane that is orthogonal to the axis of the unformed second leg. In particular, wall (560) and then wall (558) will deflect the second leg radially outwardly away from the central axis of anvil (500). Thus, staple forming pocket (550) will ultimately deflect a second leg of a staple (90) proximally and radially outwardly. Wall (556) will restrict the degree to which the second leg of staple (90) deflects radially outwardly.

Each staple forming pocket (570) comprises a staple entry surface (572) and a staple exit surface (574). Surfaces (572, 574) are contiguous with each other and define a concave recess. The concave recess formed by surfaces (572, 574) is further defined by an inner wall (576), a first outer wall (578), a second outer wall (580), and a third outer wall (582). In the present example, walls (576, 578, 580, 582) are each substantially flat. Wall (578) defines a relatively narrow, tapered gap with wall (576). Wall (582) defines a relatively wide gap with wall (576). Wall (580) is obliquely angled, providing an inwardly sloped transition from wall (582) to wall (578). Thus, walls (578, 580, 582) together provide a dogleg configuration. The edge connecting wall (576) with wall (582) is substantially straight in this example. Similarly, the edge connecting wall (576) with wall (578) is substantially straight in this example.

It should be understood that when a first leg of staple (90) is driven into staple forming pocket (570), the first leg first encounters staple entry surface (572), bends generally toward the second leg of staple (90) along a first plane that is orthogonal to the axis of the unformed first leg, and then bends proximally back generally toward the crown of staple (90). In addition, the first leg will eventually encounter wall (580), which will provide a cam surface bending the first leg along a second plane that is orthogonal to the axis of the unformed first leg. In particular, wall (580) and then wall (578) will deflect the first leg radially inwardly toward the central axis of anvil (500). Thus, staple forming pocket (570) will ultimately deflect a first leg of a staple (90) proximally and radially inwardly. Wall (576) will restrict the degree to which the first leg of staple (90) deflects radially inwardly.

As best seen in FIG. 8, staple forming pockets (510, 530, 550, 570) are arranged such that a radius line (R_{L}) extending outwardly from the center of anvil (500) passes through the region of entry surface (512) of staple forming pocket (510) and through the region of entry surface (552) of staple forming pocket (550). Thus, staple forming pockets (510, 550) overlap along a radial dimension. In addition, another radius line (R_{L}) extending outwardly from the center of anvil (500) passes through the region of entry surface (532) of staple forming pocket (530) and through the region of entry surface (572) of staple forming pocket (570). Thus, staple forming pockets (530, 570) overlap along a radial dimension. In addition, another radius line (R_{L}) extending outwardly from the center of anvil (500) passes through the region of exit surface (574) of staple forming pocket (570) and through the region of exit surface (554) of staple forming pocket (550). Thus, staple forming pockets (550, 570) overlap along a radial dimension. It should also be understood that staple forming pockets (550, 570) in each pair of pockets (550, 570) are interlocking in this configuration. In addition, another radius line (R_{L}) extending outwardly from the center of anvil (500) passes through the region of exit surface (514) of staple forming pocket (510) and through the region of exit surface (534) of staple forming pocket (530). Thus, staple forming pockets (510, 530) overlap along a radial dimension. It should also be understood that staple forming pockets (510, 530) in each pair of pockets (510, 530) are interlocking in this configuration.

In the present example, inner array (502) and outer array (504) are configured similarly, such that the inner-most pocket (510) in each pair of inner pockets (510, 530) is on the left-hand side (in the view of FIG. 9) of the pair of pockets (510, 530); and such that the inner-most pocket (570) in each pair of outer pockets (550, 570) is on the left-hand side (in the view of FIG. 9) of the pair of pockets (550, 570).

Also in the present example, the end of wall (536) associated with staple entry surface (532) includes a bent region (537), which bends slightly inwardly toward the central region of anvil (500). It should be understood that this bent region (537) may be formed in order to maintain a minimum distance between wall (536) and wall (576), thereby maintaining a minimum distance between staple forming pocket (530) and staple forming pocket (570), which may further provide more reliable manufacturing of anvil (600). In addition, bent region (537) may provide different behavior of the second leg of the staple (90) that is formed by staple forming pocket (530). Such different behavior may relate to deflections in anvil (500) and/or a tilt that might result in the first and second legs of a given staple (90) contacting corresponding surfaces (512, 532) at different times during actuation of stapling head assembly (300).

It should also be understood that the presence of bent region (537) provides staple forming pocket (530) with a structural configuration that makes staple forming pocket (530) unique relative to the other staple forming pockets (510, 550, 570). By contrast, the structural configuration of staple forming pocket (510) is identical to the structural configuration of staple forming pocket (570); while the structural configuration of staple forming pocket (550) is the mirrored inverse of the structural configuration of staple forming pockets (510, 570).

In the present example, the spacing between pockets (510, 530) in each pair of pockets (510, 530) is equal to the spacing between pockets (550, 570) in each pair of pockets (550, 570). In some other versions, however, the spacing between pockets (510, 530) in each pair of pockets (510, 530) is smaller than the spacing between pockets (550, 570) in each pair of pockets (550, 570). In such versions, pockets (550, 570) may be used to form staples (90) having a longer crown width than the crown width of staples (90) that are formed using pockets (510, 530). As another merely illustrative variations, the spacing between pockets (510, 530) in each pair of pockets (510, 530) may be larger than the spacing between pockets (550, 570) in each pair of pockets (550, 570). In such versions, pockets (550, 570) may be used to form staples (90) having a shorter crown width than the crown width of staples (90) that are formed using pockets (510, 530).

As also seen in FIG. 9, staple forming pockets (510, 530) are arranged such that they are not fully centered along a circumferential line (C_{L}) extending along surface (506) at a constant radius from the center of anvil (500). The outermost regions of staple entry surfaces (512, 532) are radially centered along the same circumferential line (C_{L}). However, staple forming pocket (510) is oriented substantially obliquely relative to circumferential line (C_{L}), such that staple exit surface (514) is positioned substantially radially inwardly from circumferential line (C_{L}). By contrast, staple exit surface (534) is positioned substantially along, with a portion position slightly radially outwardly from, circumferential line (C_{L}). In other words, while staple forming pocket (530) is substantially aligned along circumferential line (C_{L}), staple forming pocket (510) is substantially tilted radially inwardly relative to circumferential line (C_{L}), with the outermost regions of staple entry surfaces (512, 532) being radially centered along a circumferential line (C_{L}).

While the views depicted in FIGS. 9-10 only show a portion of the full circumference of anvil (500), it should be understood that the structures depicted in FIGS. 9-10 extend along the full circumference of anvil (500). The views of FIGS. 9-10 are simply being provided as an enlargement to show the structure in further detail, and are not intended to suggest that the depicted structures are only located in a limited angular range along the circumference of anvil (500).

### B. Exemplary Anvil with Symmetric Arrays of Staple Forming Pockets having Staple Leg Deflection Wall with Dogleg Configuration and Double-Chamfered Corner and Rounded Entry

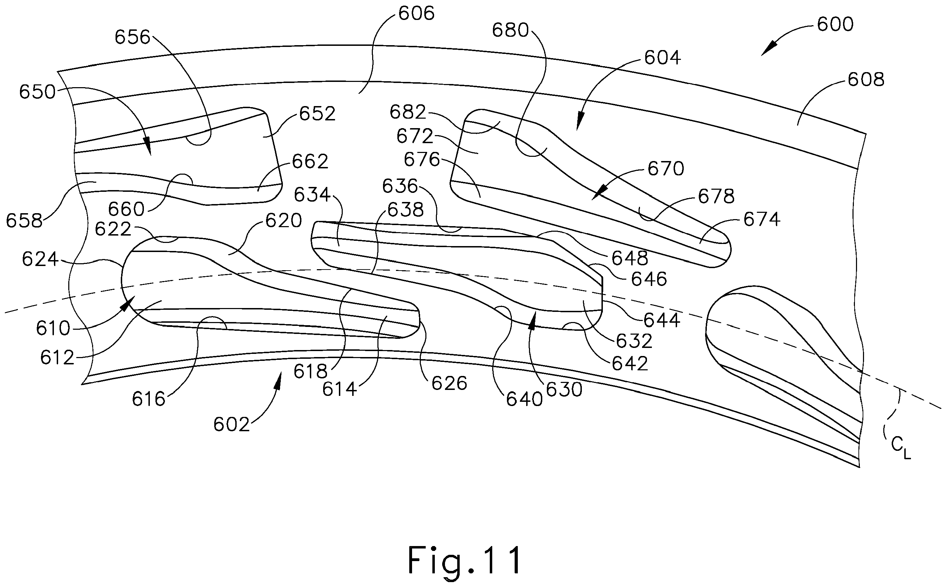

FIG. 11 shows another exemplary alternative anvil (600) that may be used with a modified version of instrument (10). Anvil (600) of this example is configured and operable just like anvil (400), except for the differences described below. Anvil (600) of the present example comprises a proximal surface (606) that defines an inner annular array (602) of staple forming pockets (610, 630) and an outer annular array (604) of staple forming pockets (650, 670). A chamfered edge (608) extends about the outer perimeter of proximal surface (606). It should be understood that anvil (600) may be secured to trocar (330), that proximal surface (606) may be used to compress tissue against deck surface (322), and that staple driver (352) may drive staples (90) through tissue into staple forming pockets (610, 630, 650, 670) in order to thereby form staples (90) in the tissue.

In the example shown in FIG. 11, each staple forming pocket (610) comprises a staple entry surface (612) and a staple exit surface (614). Surfaces (612, 614) are contiguous with each other and define a concave recess. The concave recess formed by surfaces (612, 614) is further defined by an inner wall (616), a first outer wall (618), a second outer wall (620), and a third outer wall (622). In the present example, walls (616, 618, 620, 622) are each substantially flat. Wall (618) defines a relatively narrow, tapered gap with wall (616). Wall (622) defines a relatively wide gap with wall (616). Wall (620) is obliquely angled, providing an inwardly sloped transition from wall (622) to wall (618). Thus, walls (618, 620, 622) together provide a dogleg configuration. The edge (624) connecting wall (616) with wall (622) is substantially round in this example. Similarly, the edge (626) connecting wall (616) with wall (618) is substantially round in this example. Thus, staple forming pocket (610) differs from staple forming pocket (510) in that edges (624, 626) of staple forming pocket (610) are round; while the same edges in staple forming pocket (510) are substantially straight.

It should be understood that when a first leg of staple (90) is driven into staple forming pocket (610), the first leg first encounters staple entry surface (612), bends generally toward the second leg of staple (90) along a first plane that is orthogonal to the axis of the unformed first leg, and then bends proximally back generally toward the crown of staple (90). In addition, the first leg will eventually encounter wall (620), which will provide a cam surface bending the first leg along a second plane that is orthogonal to the axis of the unformed first leg. In particular, wall (620) and then wall (618) will deflect the first leg radially inwardly toward the central axis of anvil (600). Thus, staple forming pocket (610) will ultimately deflect a first leg of a staple (90) proximally and radially inwardly. Wall (616) will restrict the degree to which the first leg of staple (90) deflects radially inwardly.

Each staple forming pocket (630) comprises a staple entry surface (632) and a staple exit surface (634). Surfaces (632, 634) are contiguous with each other and define a concave recess. The concave recess formed by surfaces (632, 634) is further defined by an outer wall (636), a first inner wall (638), a second inner wall (640), and a third inner wall (642). In the present example, walls (636, 638, 640, 642) are each substantially flat. Wall (638) defines a relatively narrow, tapered gap with wall (636). Wall (642) defines a relatively wide gap with wall (636). Wall (640) is obliquely angled, providing an outwardly sloped transition from wall (642) to wall (638). Thus, walls (638, 640, 642) together provide a dogleg configuration.

It should be understood that when a second leg of staple (90) is driven into staple forming pocket (630), the second leg first encounters staple entry surface (632), bends generally toward the first leg of staple (90) along a first plane that is orthogonal to the axis of the unformed second leg, and then bends proximally back generally toward the crown of staple (90). In addition, the second leg will eventually encounter wall (640), which will provide a cam surface bending the second leg along a second plane that is orthogonal to the axis of the unformed second leg. In particular, wall (640) and then wall (638) will deflect the second leg radially outwardly away from the central axis of anvil (600). Thus, staple forming pocket (630) will ultimately deflect a second leg of a staple (90) proximally and radially outwardly. Wall (636) will restrict the degree to which the second leg of staple (90) deflects radially outwardly.

Each staple forming pocket (650) comprises a staple entry surface (652) and a staple exit surface (654). Surfaces (652, 654) are contiguous with each other and define a concave recess. The concave recess formed by surfaces (652, 654) is further defined by an outer wall (656), a first inner wall (658), a second inner wall (660), and a third inner wall (662). In the present example, walls (656, 658, 660, 662) are each substantially flat. Wall (658) defines a relatively narrow, tapered gap with wall (656). Wall (662) defines a relatively wide gap with wall (656). Wall (660) is obliquely angled, providing an outwardly sloped transition from wall (662) to wall (658). Thus, walls (658, 660, 662) together provide a dogleg configuration. The edge connecting wall (656) with wall (662) is substantially straight in this example. Similarly, the edge connecting wall (656) with wall (658) is substantially straight in this example.

It should be understood that when a second leg of staple (90) is driven into staple forming pocket (650), the second leg first encounters staple entry surface (652), bends generally toward the first leg of staple (90) along a first plane that is orthogonal to the axis of the unformed second leg, and then bends proximally back generally toward the crown of staple (90). In addition, the second leg will eventually encounter wall (660), which will provide a cam surface bending the second leg along a second plane that is orthogonal to the axis of the unformed second leg. In particular, wall (660) and then wall (658) will deflect the second leg radially outwardly away from the central axis of anvil (600). Thus, staple forming pocket (650) will ultimately deflect a second leg of a staple (90) proximally and radially outwardly. Wall (656) will restrict the degree to which the second leg of staple (90) deflects radially outwardly.

Each staple forming pocket (670) comprises a staple entry surface (672) and a staple exit surface (674). Surfaces (672, 674) are contiguous with each other and define a concave recess. The concave recess formed by surfaces (672, 674) is further defined by an inner wall (676), a first outer wall (678), a second outer wall (680), and a third outer wall (682). In the present example, walls (676, 678, 680, 682) are each substantially flat. Wall (678) defines a relatively narrow, tapered gap with wall (676). Wall (682) defines a relatively wide gap with wall (676). Wall (680) is obliquely angled, providing an inwardly sloped transition from wall (682) to wall (678). Thus, walls (678, 680, 682) together provide a dogleg configuration. The edge connecting wall (676) with wall (682) is substantially straight in this example. Similarly, the edge connecting wall (676) with wall (678) is substantially straight in this example.

It should be understood that when a first leg of staple (90) is driven into staple forming pocket (670), the first leg first encounters staple entry surface (672), bends generally toward the second leg of staple (90) along a first plane that is orthogonal to the axis of the unformed first leg, and then bends proximally back generally toward the crown of staple (90). In addition, the first leg will eventually encounter wall (680), which will provide a cam surface bending the first leg along a second plane that is orthogonal to the axis of the unformed first leg. In particular, wall (680) and then wall (678) will deflect the first leg radially inwardly toward the central axis of anvil (600). Thus, staple forming pocket (670) will ultimately deflect a first leg of a staple (90) proximally and radially inwardly. Wall (676) will restrict the degree to which the first leg of staple (90) deflects radially inwardly.

While FIG. 11 only shows a portion of anvil (600), it should be understood that staple forming pockets (610, 630, 650, 670) may span about the full circumference of proximal surface (606), in an arrangement like staple forming pockets (510, 530, 550, 570) as shown in FIG. 8. Thus, staple forming pockets (610, 630, 650, 670) may have the same kind of overlap along a radial dimension as described above in the context of staple forming pockets (510, 530, 550, 570). In other words, while the view depicted in FIG. 11 only shows a portion of the full circumference of anvil (600), it should be understood that the structures depicted in FIG. 11 extend along the full circumference of anvil (600). The view of FIG. 11 is simply being provided as an enlargement to show the structure in further detail, and is not intended to suggest that the depicted structures are only located in a limited angular range along the circumference of anvil (600).

It should also be understood that in anvil (600), inner array (602) and outer array (604) are configured similarly, such that the inner-most pocket (610) in each pair of inner pockets (610, 630) is on the left-hand side (in the view of FIG. 11) of the pair of pockets (610, 630); and such that the inner-most pocket (670) in each pair of outer pockets (650, 670) is on the left-hand side (in the view of FIG. 11) of the pair of pockets (650, 670).

In the present example, staple forming pocket (630) is unique relative to the other staple forming pockets (610, 650, 670) of anvil (600) in that staple forming pocket (630) further includes a double-chamfered edge at the right-hand end (in the view of FIG. 11) of wall (636). In particular, this double-chamfered edge is formed by a first edge (648) that extends obliquely from wall (636); and a second edge (646) that extends obliquely from first edge (648). Second edge (646) is joined to wall (642) via a substantially flat edge (644). It should be understood that this double-chamfered edge formed by edges (646, 648) may serve a purpose similar to that described above in the context of bent region (537) of staple forming pocket (530). In particular, the configuration of edges (646, 648) may be formed in order to maintain a minimum distance between wall (636) and wall (676), thereby maintaining a minimum distance between staple forming pocket (630) and staple forming pocket (670), which may further provide more reliable manufacturing of anvil (600). In addition, the configuration of edges (646, 648) may provide different behavior of the second leg of the staple (90) that is formed by staple forming pocket (630). Such different behavior may relate to deflections in anvil (600) and/or a tilt that might result in the first and second legs of a given staple (90) contacting corresponding surfaces (612, 632) at different times during actuation of stapling head assembly (300).

As also seen in FIG. 11, staple forming pockets (610, 630) are arranged such that they are not fully centered along a circumferential line (C_{L}) extending along surface (606) at a constant radius from the center of anvil (600). The outermost regions of staple entry surfaces (612, 632) are radially centered along the same circumferential line (C_{L}). However, staple forming pocket (610) is oriented substantially obliquely relative to circumferential line (C_{L}), such that staple exit surface (614) is positioned substantially radially inwardly from circumferential line (C_{L}). By contrast, staple exit surface (634) is positioned substantially along, with a portion position slightly radially outwardly from, circumferential line (C_{L}). In other words, while staple forming pocket (630) is substantially aligned along circumferential line (C_{L}), staple forming pocket (510) is substantially tilted radially inwardly relative to circumferential line (C_{L}), with the outermost regions of staple entry surfaces (612, 632) being radially centered along a circumferential line (C_{L}).

### C. Exemplary Anvil with Inversed Arrays of Staple Forming Pockets and Overlapping Formed Leg Configuration with Lateral Leg Deflection

FIGS. 12-14 show an exemplary alternative anvil (700) that may be used with a modified version of instrument (10). Anvil (700) of this example is configured and operable just like anvil (400), except for the differences described below. Anvil (700) of the present example comprises a proximal surface (706) that defines an inner annular array (702) of staple forming pockets (710, 730) and an outer annular array (704) of staple forming pockets (750, 770). A chamfered edge (708) extends about the outer perimeter of proximal surface (706). It should be understood that anvil (700) may be secured to trocar (330), that proximal surface (706) may be used to compress tissue against deck surface (322), and that staple driver (352) may drive staples (90) through tissue into staple forming pockets (710, 730, 750, 770) in order to thereby form staples (90) in the tissue.

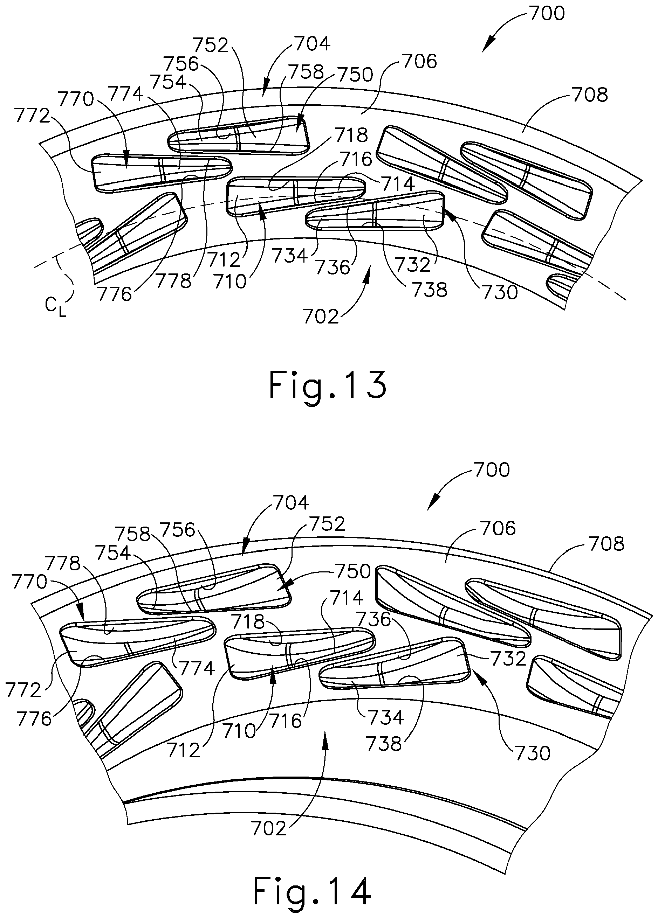

As best seen in FIGS. 13-14, each staple forming pocket (710) comprises a staple entry surface (712) and a staple exit surface (714). Surfaces (712, 714) are contiguous with each other and define a concave recess. The concave recess formed by surfaces (712, 714) is further defined by an inner wall (716) and an outer wall (718). In the present example, walls (716, 718) are each substantially flat. Walls (716, 718) together define a taper, such that the gap between walls (716, 718) is smaller near exit surface (714) than the gap between walls (716, 718) at entry surface (712).

It should be understood that when a first leg of staple (90) is driven into staple forming pocket (710), the first leg first encounters staple entry surface (712), bends generally toward the second leg of staple (90) along a first plane that is orthogonal to the axis of the unformed first leg, and then bends proximally back generally toward the crown of staple (90). In addition, the first leg will eventually encounter wall (716), which will provide a cam surface bending the first leg along a second plane that is orthogonal to the axis of the unformed first leg. In particular, wall (716) will deflect the first leg radially outwardly away from the central axis of anvil (700). Thus, staple forming pocket (710) will ultimately deflect a first leg of a staple (90) proximally and radially outwardly. Wall (718) will restrict the degree to which the first leg of staple (90) deflects radially outwardly.

Each staple forming pocket (730) comprises a staple entry surface (732) and a staple exit surface (734). Surfaces (732, 734) are contiguous with each other and define a concave recess. The concave recess formed by surfaces (732, 734) is further defined by an inner wall (738) and an outer wall (736). In the present example, walls (736, 738) are each substantially flat. Walls (736, 738) together define a taper, such that the gap between walls (736, 738) is smaller near exit surface (734) than the gap between walls (736, 738) at entry surface (732).

It should be understood that when a second leg of staple (90) is driven into staple forming pocket (730), the second leg first encounters staple entry surface (732), bends generally toward the first leg of staple (90) along a first plane that is orthogonal to the axis of the unformed second leg, and then bends proximally back generally toward the crown of staple (90). In addition, the second leg will eventually encounter wall (736), which will provide a cam surface bending the second leg along a second plane that is orthogonal to the axis of the unformed second leg. In particular, wall (736) will deflect the second leg radially inwardly toward the central axis of anvil (700). Thus, staple forming pocket (730) will ultimately deflect a second leg of a staple (90) proximally and radially inwardly. Wall (738) will restrict the degree to which the second leg of staple (90) deflects radially inwardly.

Each staple forming pocket (750) comprises a staple entry surface (752) and a staple exit surface (754). Surfaces (752, 754) are contiguous with each other and define a concave recess. The concave recess formed by surfaces (752, 754) is further defined by an inner wall (758) and an outer wall (756). In the present example, walls (756, 758) are each substantially flat. Walls (756, 758) together define a taper, such that the gap between walls (756, 758) is smaller near exit surface (754) than the gap between walls (756, 758) at entry surface (752).

It should be understood that when a second leg of staple (90) is driven into staple forming pocket (750), the second leg first encounters staple entry surface (752), bends generally toward the first leg of staple (90) along a first plane that is orthogonal to the axis of the unformed second leg, and then bends proximally back generally toward the crown of staple (90). In addition, the second leg will eventually encounter wall (758), which will provide a cam surface bending the second leg along a second plane that is orthogonal to the axis of the unformed second leg. In particular, wall (758) will deflect the second leg radially outwardly away from the central axis of anvil (700). Thus, staple forming pocket (750) will ultimately deflect a second leg of a staple (90) proximally and radially outwardly. Wall (756) will restrict the degree to which the second leg of staple (90) deflects radially outwardly.

Each staple forming pocket (770) comprises a staple entry surface (772) and a staple exit surface (774). Surfaces (772, 774) are contiguous with each other and define a concave recess. The concave recess formed by surfaces (772, 774) is further defined by an inner wall (776) and an outer wall (778). In the present example, walls (776, 778) are each substantially flat. Walls (776, 778) together define a taper, such that the gap between walls (776, 778) is smaller near exit surface (774) than the gap between walls (776, 778) at entry surface (772).

It should be understood that when a first leg of staple (90) is driven into staple forming pocket (770), the first leg first encounters staple entry surface (772), bends generally toward the second leg of staple (90) along a first plane that is orthogonal to the axis of the unformed first leg, and then bends proximally back generally toward the crown of staple (90). In addition, the first leg will eventually encounter wall (778), which will provide a cam surface bending the first leg along a second plane that is orthogonal to the axis of the unformed first leg. In particular, wall (778) will deflect the first leg radially inwardly toward the central axis of anvil (700). Thus, staple forming pocket (770) will ultimately deflect a first leg of a staple (90) proximally and radially inwardly. Wall (776) will restrict the degree to which the first leg of staple (90) deflects radially inwardly.

As best seen in FIG. 12, staple forming pockets (710, 730, 750, 770) are arranged such that a radius line (R_{L}) extending outwardly from the center of anvil (700) passes through the region of exit surface (774) of staple forming pocket (770) and through the region of exit surface (754) of staple forming pocket (750). Thus, staple forming pockets (750, 770) overlap along a radial dimension. It should also be understood that staple forming pockets (750, 770) in each pair of pockets (750, 770) are interlocking in this configuration. In addition, another radius line (R_{L}) extending outwardly from the center of anvil (700) passes through the region of exit surface (714) of staple forming pocket (710) and through the region of exit surface (734) of staple forming pocket (730). Thus, staple forming pockets (710, 730) overlap along a radial dimension. It should also be understood that staple forming pockets (710, 730) in each pair of pockets (710, 730) are interlocking in this configuration. In addition, another radius line (R_{L}) extending outwardly from the center of anvil (700) passes through the region of entry surface (732) of staple forming pocket (730) and through the region of entry surface (772) of staple forming pocket (770). Thus, staple forming pockets (730, 770) overlap along a radial dimension. In addition, another radius line (R_{L}) extending outwardly from the center of anvil (700) passes through the region of entry surface (712) of staple forming pocket (710) and through the region of entry surface (752) of staple forming pocket (750). Thus, staple forming pockets (710, 750) overlap along a radial dimension.

In the present example, inner array (702) and outer array (704) are arranged to provide mirrored symmetry, such that the outer-most pocket (710) in each pair of inner pockets (710, 730) is on the left-hand side (in the view of FIGS. 13-14) of the pair of pockets (710, 730); and such that the inner-most pocket (770) in each pair of outer pockets (750, 770) is on the left-hand side (in the view of FIGS. 13-14) of the pair of pockets (750, 770). Thus, the symmetry between arrays (702, 704) is opposite of the symmetry between arrays (502, 504) described above.

As also seen in FIG. 13, staple forming pockets (710, 730) are arranged such that they are substantially centered along a circumferential line (C_{L}) extending along surface (706) at a constant radius from the center of anvil (700). The outermost regions of staple entry surfaces (712, 732) are radially centered along the same circumferential line (C_{L}). In addition, circumferential line (C_{L}) intersects wall (716) at a point along the length of wall (716) that is at the same point along the length of wall (736) at which circumferential line (C_{L}) intersects wall (736). Thus, as a pair, staple forming pockets (710, 730) are radially centered along circumferential line (C_{L}).

While the views depicted in FIGS. 13-14 only show a portion of the full circumference of anvil (700), it should be understood that the structures depicted in FIGS. 13-14 extend along the full circumference of anvil (700). The views of FIGS. 13-14 are simply being provided as an enlargement to show the structure in further detail, and are not intended to suggest that the depicted structures are only located in a limited angular range along the circumference of anvil (700).

### D. Exemplary Anvil with Inner Array of Offset Pockets and Outer Array of Aligned Pockets

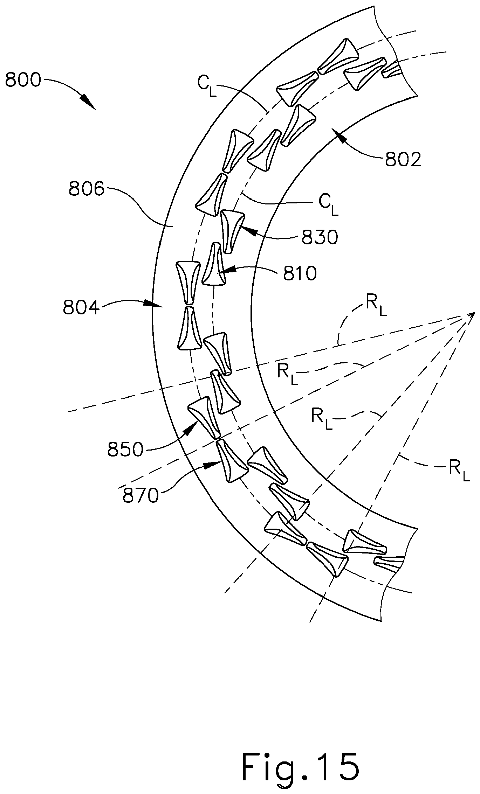

FIG. 15 shows another exemplary alternative anvil (800) that may be used with a modified version of instrument (10). Anvil (800) of this example is configured and operable just like anvil (400), except for the differences described below. Anvil (800) of the present example comprises a proximal surface (806) that defines an inner annular array (802) of staple forming pockets (810, 830) and an outer annular array (804) of staple forming pockets (850, 870). In some versions, a chamfered edge (not shown) extends about the outer perimeter of proximal surface (806). It should be understood that anvil (800) may be secured to trocar (330), that proximal surface (806) may be used to compress tissue against deck surface (322), and that staple driver (352) may drive staples (880, 890) through tissue into staple forming pockets (810, 830, 850, 870) in order to thereby form staples (880, 890) in the tissue.

In the present example, the structural configuration of staple forming pocket (810) is identical to the structural configuration of staple forming pocket (710), the structural configuration of staple forming pocket (830) is identical to the structural configuration of staple forming pocket (730), the structural configuration of staple forming pocket (850) is identical to the structural configuration of staple forming pocket (750), and the structural configuration of staple forming pocket (870) is identical to the structural configuration of staple forming pocket (770). Thus, the structural configurations of (810, 830, 850, 870) will not be discussed in further detail here. In addition, the positioning and arrangement of staple forming pockets (810, 830) is identical to the positioning and arrangement of staple forming pockets (710, 730). Thus, the relationship between staple forming pockets (810, 830) and a circumferential line (C_{L}) is the same as the above-noted relationship between forming pockets (710, 730) and a corresponding circumferential line (C_{L}).

Unlike staple forming pockets (750, 770), staple forming pockets (850, 870) are arranged such that they do not overlap each other. Instead, staple forming pockets (850, 870) are aligned with each other such that a circumferential line (C_{L}) is centered along the staple entry and staple exit surfaces of staple forming pockets (750, 770).

As shown in FIG. 15, staple forming pockets (810, 830, 850, 870) are arranged such that a radius line (R_{L}) extending outwardly from the center of anvil (800) passes through a staple forming pocket (830) and an adjacent staple forming pocket (870); such that another radius line (R_{L}) extending outwardly from the center of anvil (800) passes through a staple forming pocket (810) and an adjacent staple forming pocket (850); and such that another radius line (R_{L}) extending outwardly from the center of anvil (800) passes through both staple forming pockets (810, 830) in each pair of staple forming pockets (810, 830). However, no radius line (R_{L}) extending outwardly from the center of anvil (800) passes through both staple forming pockets (850, 870) in each pair of staple forming pockets (850, 870). Instead, a given radius line (R_{L}) extending outwardly from the center of anvil (800) will either pass through only one staple forming pockets (850, 870) in each pair of staple forming pockets (850, 870); or pass through no staple forming pockets (850, 870) at all. Indeed, FIG. 15 shows one radius line (R_{L}) extending outwardly from the center of anvil (800) passing between forming pockets (850, 870).

While the view depicted in FIG. 15 only shows a portion of the full circumference of anvil (800), it should be understood that the structures depicted in FIG. 15 extend along the full circumference of anvil (800). The view of FIG. 15 is simply being provided as an enlargement to show the structure in further detail, and is not intended to suggest that the depicted structures are only located in a limited angular range along the circumference of anvil (800).

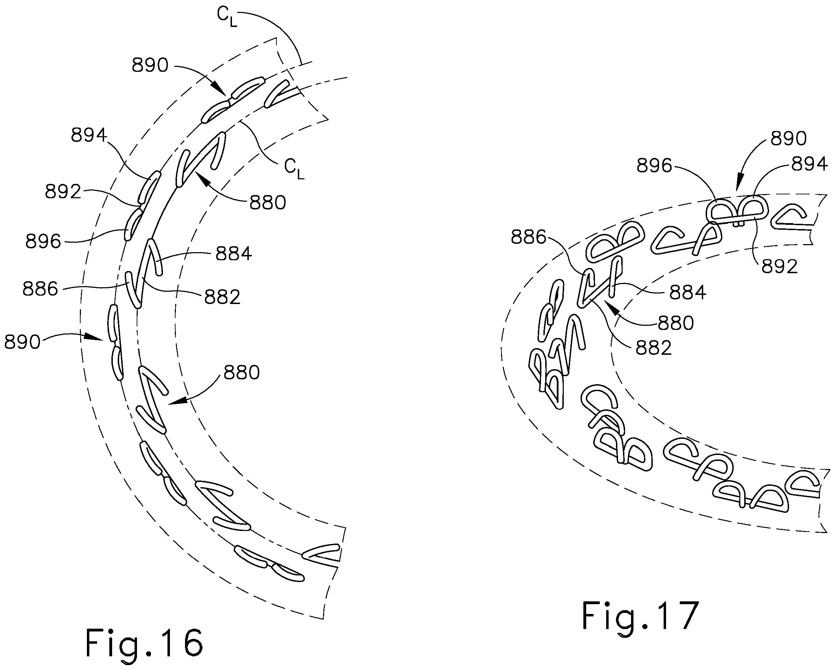

FIGS. 16-17 show two arrays of staples (880, 890) that have been formed using anvil (800). In particular, FIGS. 16-17 show an inner array of staples (880) formed by staple forming pockets (810, 830) and an outer array of staples (890) formed by staple forming pockets (850, 870). As shown, each outer staple (890) includes a crown (892) and an associated pair of bent legs (894, 896). Since the staple entry and exit regions of staple forming pockets (850, 870) are centered along a circumferential line (C_{L}), the crowns (892) and bent legs (894, 896) are also centered along a circumferential line (C_{L}). It should therefore be understood that formed staples (890) are substantially two-dimensional-extending along a circumferential dimension (i.e., along circumferential line (C_{L})) and extending along a longitudinal dimension (i.e., into and out of the page in the view of FIG. 16). This two-dimensional configuration of formed staples (890) provides an appearance similar to the letter "B."

By contrast, each inner staple (880) includes bent legs (884, 886) that are deflected off-plane from the corresponding crown (882). In particular, while crowns (882) are substantially centered along circumferential line (C_{L}), each bent leg (884) is deflected radially inwardly relative to circumferential line (C_{L}) while each bent leg (886) is deflected radially outwardly relative to circumferential line (C_{L}). This configuration is due to the offset relationship between staple forming pockets (810, 830) and a corresponding circumferential line (C_{L}). It should be understood that staples formed by staple forming pockets (510, 530, 550, 570, 610, 630, 650, 670, 710, 730, 750, 770) described above may also have a configuration that is similar to formed staples (880). It should also be understood that formed staples (880) are substantially three-dimensional-extending along a circumferential dimension (i.e., along circumferential line (C_{L})), extending along a longitudinal dimension (i.e., into and out of the page in the view of FIG. 16), and extending along a radial dimension.

### E. Exemplary Anvil with Inversed Arrays of Staple Forming Pockets and Non-Overlapping Formed Leg Configuration with Lateral Leg Deflection

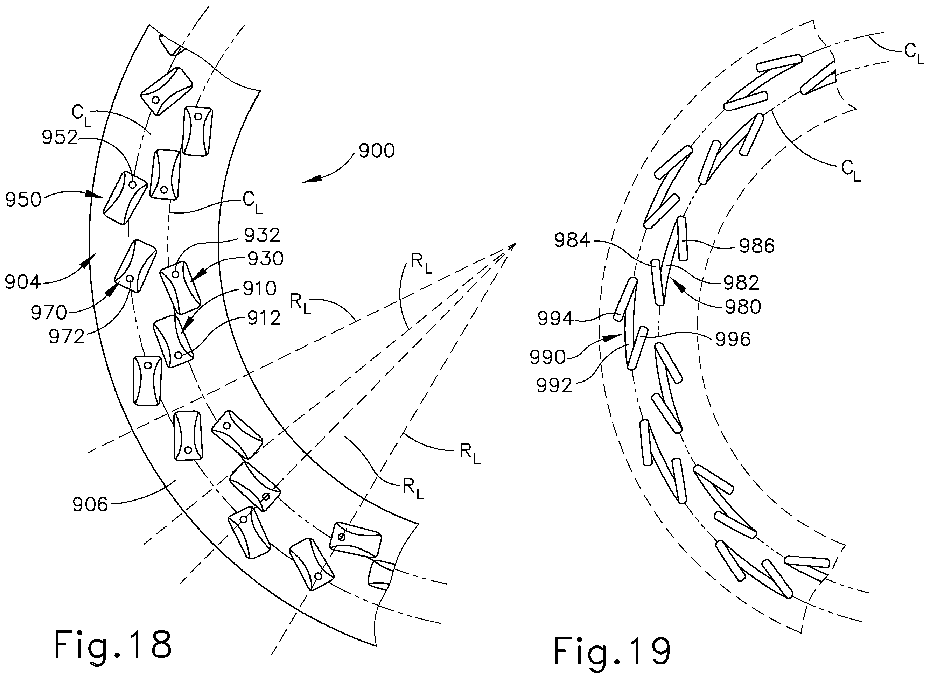

FIG. 18 shows another exemplary alternative anvil (900) that may be used with a modified version of instrument (10). Anvil (900) of this example is configured and operable just like anvil (400), except for the differences described below. Anvil (800) of the present example comprises a proximal surface (906) that defines an inner annular array (902) of staple forming pockets (910, 930) and an outer annular array (904) of staple forming pockets (950, 970). In some versions, a chamfered edge (not shown) extends about the outer perimeter of proximal surface (906). It should be understood that anvil (900) may be secured to trocar (330), that proximal surface (906) may be used to compress tissue against deck surface (322), and that staple driver (352) may drive staples (980, 990) through tissue into staple forming pockets (910, 930, 950, 970) in order to thereby form staples (980, 990) in the tissue.

In the present example, all staple forming pockets (910, 930, 950, 970) are configured identically to each other. In particular, each staple forming pocket (910, 930, 950, 970) has a generally rectangular shape at proximal surface (906), such that staple forming pockets (910, 930, 950, 970) are not tapered along proximal surface (906). However, the sidewalls of staple forming pockets (910, 930, 950, 970) slope toward each other as the sidewalls approach the floors of staple forming pockets (910, 930, 950, 970). The floors of staple forming pockets (910, 930, 950, 970) are concave, such that a staple leg (984, 986, 994, 996) will first encounter a corresponding staple entry point (912, 932, 952, 972), then bend generally toward the other leg of staple (980, 990) along a first plane that is orthogonal to the axis of the unformed first leg, and then bend proximally back generally toward the crown (982, 992) of staple (980, 990).

As also seen in FIG. 18, each pair of staple forming pockets (910, 930) is centered along a corresponding circumferential line (C_{L}), and each pair of staple forming pockets (950, 970) is also centered along a corresponding circumferential line (C_{L}). In particular, an inner circumferential line (C_{L}) passes through each staple entry point (912, 932); while an outer circumferential line (C_{L}) passes through each staple entry point (952, 972). While staple forming pockets (910, 930, 950, 970), as pairs, are centered along corresponding circumferential lines (C_{L}), each staple forming pocket (910, 930, 950, 970) is oriented obliquely relative to the corresponding circumferential line (C_{L}). In addition, each staple forming pocket (910) is angularly offset relative to the other staple forming pocket (930) in the pair of pockets (910, 930); and each staple forming pocket (950) is angularly offset relative to the other staple forming pocket (970) in the pair of pockets (950, 970).

It should be understood from the foregoing that, in addition to bending legs (984, 986, 994, 996) back toward corresponding crowns (982, 992) along respective first planes that are orthogonal to the respective axes of the respective unformed first legs (984, 986, 994, 996), staple forming pockets (910, 930, 950, 970) will bend legs (984, 986, 994, 996) along respective second planes that are orthogonal to the respective axes of the respective unformed first legs (984, 986, 994, 996). In particular, staple forming pocket (910) will bend leg (984) radially outwardly away from the central axis of anvil (900); staple forming pocket (930) will bend leg (986) radially inwardly toward the central axis of anvil (900); staple forming pocket (950) will bend leg (994) radially outwardly away from the central axis of anvil (900); and staple forming pocket (970) will bend leg (996) radially inwardly toward the central axis of anvil (900).

In the present example, inner array (902) and outer array (904) are arranged to provide mirrored symmetry, such that the pocket (910) in each pair of inner pockets (910, 930) providing the outer-most formed leg (984) is on the bottom (in the view of FIG. 18) of the pair of pockets (910, 930); and such that pocket (970) in each pair of outer pockets (950, 970) providing the inner-most formed leg (996) is on the bottom (in the view of FIG. 18) of the pair of pockets (950, 970). Conversely, the pocket (930) in each pair of inner pockets (910, 930) providing the inner-most formed leg (986) is on the top (in the view of FIG. 18) of the pair of pockets (910, 930); and the pocket (950) in each pair of outer pockets (950, 970) providing the outer-most formed leg (994) is on the top (in the view of FIG. 18) of the pair of pockets (950, 970). Thus, the symmetry between arrays (902, 904) is opposite of the symmetry between arrays (502, 504) described above. Instead, the symmetry between arrays (902, 904) is like the symmetry between arrays (702, 704).

As also seen in FIG. 18, staple forming pockets (910, 930, 950, 970) are arranged such that a radius line (R_{L}) extending outwardly from the center of anvil (900) passes through the staple entry point (932) of staple forming pocket (930) and through the staple entry point (972) of staple forming pocket (970). Thus, staple forming pockets (930, 970) overlap along a radial dimension. In addition, another radius line (R_{L}) extending outwardly from the center of anvil (900) passes through the staple entry point (912) of staple forming pocket (910) and through the staple entry point (952) of staple forming pocket (950). Thus, staple forming pockets (910, 970) overlap along a radial dimension. In addition, another radius line (R_{L}) extending outwardly from the center of anvil (900) passes between staple forming pockets (910, 930) such that staple forming pockets (910, 930) in each pair of staple forming pockets (910, 930) do not overlap each other along a radial dimension. Similarly, another radius line (R_{L}) extending outwardly from the center of anvil (900) passes between staple forming pockets (950, 970) such that staple forming pockets (950, 970) in each pair of staple forming pockets (950, 970) do not overlap each other along a radial dimension.

While the view depicted in FIG. 18 only shows a portion of the full circumference of anvil (900), it should be understood that the structures depicted in FIG. 18 extend along the full circumference of anvil (900). The view of FIG. 18 is simply being provided as an enlargement to show the structure in further detail, and is not intended to suggest that the depicted structures are only located in a limited angular range along the circumference of anvil (900).

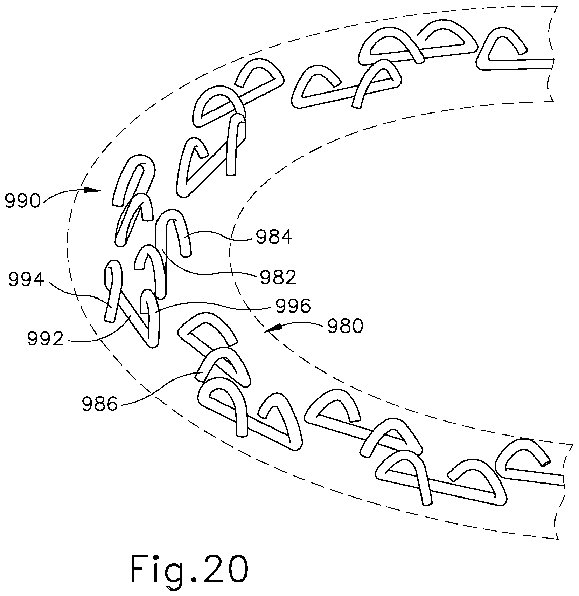

FIGS. 19-20 show two annular arrays of staples (980, 990) that have been formed using anvil (900). As shown, crowns (982) are all substantially aligned along the inner circumferential line (C_{L}); while crowns (992) are all substantially aligned along the outer circumferential line (C_{L}). FIGS. 19-20 also show how legs (984, 994) are outwardly deflected relative to corresponding circumferential lines (C_{L}); and how legs (986, 996) are inwardly deflected relative to corresponding circumferential lines (C_{L}). It should therefore be understood that formed staples (980, 990) are substantially three-dimensional-extending along a circumferential dimension (i.e., along circumferential line (C_{L})), extending along a longitudinal dimension (i.e., into and out of the page in the view of FIG. 19), and extending along a radial dimension.

### F. Exemplary Anvil with Inversed Arrays of Staple Forming Pockets and Non-Overlapping Formed Leg Configuration without Lateral Leg Deflection

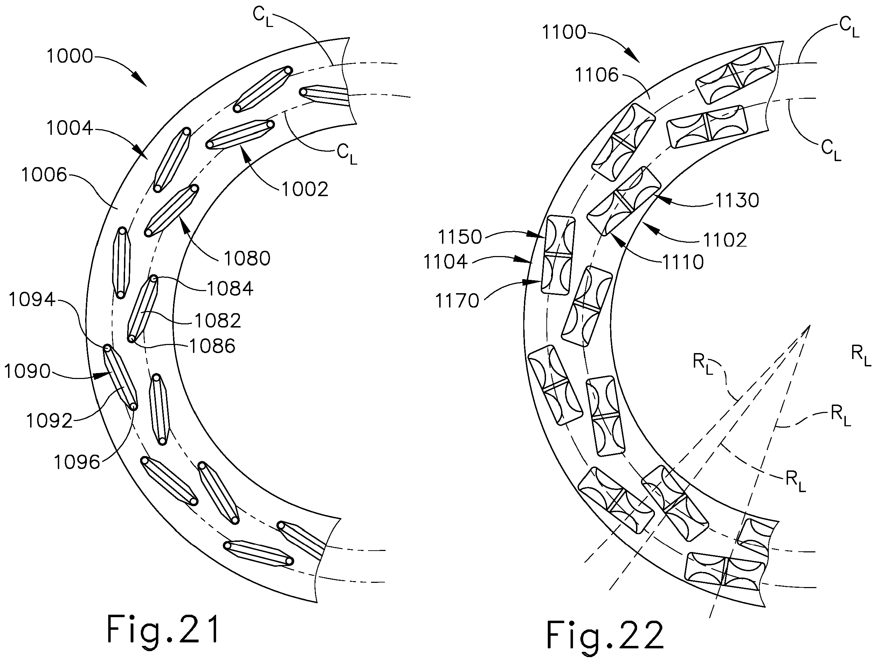

FIG. 21 shows an exemplary alternative deck member (1000), while FIG. 22 shows an exemplary alternative anvil (1100) that may be used with deck member (1000). Deck member (1000) and anvil (1100) may both be used with a modified version of instrument (10). It should be understood that deck member (1000) may be readily incorporated into stapling head assembly (300) in place of deck member (320). Deck member (1000) of this example is configured and operable just like deck member (320), except for the differences described below. Deck member (1000) of the present example comprises a deck surface (1006) defining an inner annular array of staple openings (1002) and an outer annular array of staple openings (1004). A staple (1080) is positioned in each inner staple opening (1002) while a staple (1090) is positioned in each outer staple opening (1004). It should be understood that a staple driver member (not shown), which may be configured and operable similar to staple driver member (350) described above, may be positioned under staples (1080, 1090) and may thereby drive staples (1080, 1090) out through openings (1002, 1004).

Openings (1002) are configured and arranged such that openings (1002) are oriented obliquely relative to circumferential line (C_{L}). In particular, openings (1002) are configured and arranged such that circumferential line (C_{L}) will pass through the center of each crown (1082) of each staple (1080); such that first leg (1084) of each staple (1080) is positioned radially inwardly relative to circumferential line (C_{L}); and such that second leg (1086) of each staple (1080) is positioned radially outwardly relative to circumferential line (C_{L}). Similarly, openings (1004) are configured and arranged such that openings (1004) are oriented obliquely relative to circumferential line (C_{L}). In particular, openings (1004) are configured and arranged such that circumferential line (C_{L}) will pass through the center of each crown (1092) of each staple (1090); such that first leg (1094) of each staple (1090) is positioned radially outwardly relative to circumferential line (C_{L}); and such that second leg (1096) of each staple (1090) is positioned radially inwardly relative to circumferential line (C_{L}).

Anvil (1100) of this example is configured and operable just like anvil (400), except for the differences described below. Anvil (1100) of the present example comprises a proximal surface (1106) that defines an inner annular array (1102) of staple forming pockets (1110, 1130) and an outer annular array (1104) of staple forming pockets (1150, 1170). In some versions, a chamfered edge (not shown) extends about the outer perimeter of proximal surface (106). It should be understood that anvil (1100) may be secured to trocar (330), that proximal surface (106) may be used to compress tissue against deck surface (1006), and that a staple driver (not shown) may drive staples (1080, 1090) through tissue into staple forming pockets (1110, 1130, 1150, 1170) in order to thereby form staples (1080, 1090) in the tissue.

In the present example, all staple forming pockets (1110, 1130, 1150, 170) are configured identically to each other. In particular, each staple forming pocket (1110, 1130, 1150, 1170) has a generally rectangular shape at proximal surface (1106), such that staple forming pockets (1110, 1130, 1150, 1170) are not tapered along proximal surface (1106). However, the sidewalls of staple forming pockets (1110, 1130, 1150, 1170) slope toward each other as the sidewalls approach the floors of staple forming pockets (1110, 1130, 1150, 1170). The floors of staple forming pockets (1110, 1130, 1150, 1170) are concave, such that a staple leg (1084, 1086, 1094, 1096) will first encounter a corresponding staple entry point, then bend generally toward the other leg of staple (1080, 1090) along a first plane that is orthogonal to the axis of the unformed first leg, and then bend proximally back generally toward the crown (1082, 1092) of staple (1080, 1090).

As also seen in FIG. 22, each pair of staple forming pockets (1110, 1130) is centered along a corresponding circumferential line (C_{L}), and each pair of staple forming pockets (1150, 1170) is also centered along a corresponding circumferential line (C_{L}). In particular, an inner circumferential line (C_{L}) passes through the center and opposing corners of each pair of staple forming pockets (1110, 1130); while an outer circumferential line (C_{L}) passes through the center and opposing corners of each pair of staple forming pockets (1150, 1170). While staple forming pockets (1110, 1130, 1150, 1170), as pairs, are centered along corresponding circumferential lines (C_{L}), each pair of staple forming pockets (1110, 1130, 1150, 1170) is oriented obliquely relative to the corresponding circumferential line (C_{L}). In addition, each staple forming pocket (1110) is angularly aligned with the other staple forming pocket (1130) in the pair of pockets (1110, 1130); and each staple forming pocket (1150) is angularly aligned with the other staple forming pocket (1170) in the pair of pockets (1150, 1170).

In the present example, inner array (1102) and outer array (1104) are arranged to provide mirrored symmetry, such that the pocket (1110) in each pair of inner pockets (1110, 1130) providing the outer-most formed leg (1086) is on the bottom (in the view of FIG. 22) of the pair of pockets (1110, 1130); and such that pocket (1170) in each pair of outer pockets (1150, 1170) providing the inner-most formed leg (1096) is on the bottom (in the view of FIG. 22) of the pair of pockets (1150, 1170). Conversely, the pocket (1130) in each pair of inner pockets (1110, 1130) providing the inner-most formed leg (1084) is on the top (in the view of FIG. 22) of the pair of pockets (1110, 1130); and the pocket (1150) in each pair of outer pockets (1150, 1170) providing the outer-most formed leg (1094) is on the top (in the view of FIG. 22) of the pair of pockets (1150, 1170). Thus, the symmetry between arrays (1102, 1104) is opposite of the symmetry between arrays (502, 504) described above. Instead, the symmetry between arrays (1102, 1104) is like the symmetry between arrays (702, 704) and the symmetry between arrays (902, 904).

As also seen in FIG. 22, staple forming pockets (1110, 1130, 1150, 1170) are arranged such that a radius line (R_{L}) extending outwardly from the center of anvil (1100) passes between staple forming pockets (1150, 1170) such that staple forming pockets (1150, 1170) in each pair of staple forming pockets (1150, 1170) do not overlap each other along a radial dimension. In addition, another radius line (R_{L}) extending outwardly from the center of anvil (1100) passes between staple forming pockets (1110, 1130) such that staple forming pockets (1110, 1130) in each pair of staple forming pockets (1110, 1130) do not overlap each other along a radial dimension. In addition, another radius line (R_{L}) extending outwardly from the center of anvil (1100) passes through staple forming pocket (1130) and through staple forming pocket (1170). Thus, staple forming pockets (1130, 1170) overlap along a radial dimension. It should be understood that staple forming pockets (1110, 1150) have the same kind of overlap along a radial dimension.

In the present example staples (1090, 1080) formed by anvil (1100) are only substantially two-dimensional-extending along dimension that is oblique relative to circumferential line (C_{L}) and extending along a longitudinal dimension (i.e., into and out of the page in the view of FIG. 22). This two-dimensional configuration of formed staples (1090, 1090) provides an appearance similar to the letter "B."

While the view depicted in FIG. 22 only shows a portion of the full circumference of anvil (1100), it should be understood that the structures depicted in FIG. 22 extend along the full circumference of anvil (1100). The view of FIG. 22 is simply being provided as an enlargement to show the structure in further detail, and is not intended to suggest that the depicted structures are only located in a limited angular range along the circumference of anvil (1100).

### IV. Miscellaneous

It should be understood that any one or more of the teachings, expressions, examples, etc. described herein may be combined with any one or more of the other described herein. The above-described teachings should therefore not be viewed in isolation relative to each other. Various suitable ways in which the teachings herein may be combined will be readily apparent to those of ordinary skill in the art.

At least some of the teachings herein may be readily combined with one or more teachings of U.S. Pat. No. 7,794,475, entitled "Surgical Staples Having Compressible or Crushable Members for Securing Tissue Therein and Stapling Instruments for Deploying the Same," issued September 14, 2010; U.S. Pub. No. 2014/0151429, entitled "Trans-Oral Circular Anvil Introduction System with Dilation Feature," published June 5, 2014; U.S. Pub. No. 2014/0144968, entitled "Surgical Staple with Integral Pledget for Tip Deflection," published May 29, 2014; U.S. Pub. No. 2014/0158747, entitled "Surgical Stapler with Varying Staple Widths along Different Circumferences," published June 12, 2014; U.S. Pub. No. 2014/0144969, entitled "Pivoting Anvil for Surgical Circular Stapler," published May 29, 2014; U.S. Pub. No. 2014/0151430, entitled "Circular Anvil Introduction System with Alignment Feature," published June 5, 2014; U.S. Pub. No. 2014/0166717, entitled "Circular Stapler with Selectable Motorized and Manual Control, Including a Control Ring," published June 19, 2014; U.S. Pub. No. 2014/0166728, entitled "Motor Driven Rotary Input Circular Stapler with Modular End Effector," published June 19, 2014; and/or U.S. Pub. No. 2014/0166718, entitled "Motor Driven Rotary Input Circular Stapler with Lockable Flexible Shaft," published June 19, 2014. Various suitable ways in which such teachings may be combined will be apparent to those of ordinary skill in the art.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Further adaptations of the systems described herein may be accomplished by appropriate modifications by one of ordinary skill in the art. Several such potential modifications have been mentioned, and others will be apparent to those skilled in the art. For instance, the examples, geometries, materials, dimensions, ratios, steps, and the like discussed above are illustrative and are not required. Accordingly, the scope of the present invention is defined by the following claims and is not limited to the details of structure and operation shown and described in the specification and drawings.

## Claims

1. An apparatus comprising:
(a) an anvil surface configured to compress tissue against a deck surface, wherein the anvil surface defines:
(i) an inner annular array (702; 1102) of staple forming pockets, wherein the inner annular array of staple forming pockets comprises a plurality of staple forming pocket pairs, wherein each pair of staple forming pockets in the inner annular array comprises:
(A) a first innermost staple forming pocket (730; 1130), and
(B) a second outermost staple forming pocket (710; 1110), and
(ii) an outer annular array (704; 1104) of staple forming pockets, wherein the outer annular array of staple forming pockets comprises a plurality of staple forming pocket pairs, wherein each pair of staple forming pockets in the outer annular array comprises:
(A) a first innermost staple forming pocket (770; 1170), and
(B) a second outermost staple forming pocket (750; 1150); and
(b) an anvil shaft extending along a longitudinal axis, wherein the anvil shaft is configured to couple with a stapling head assembly of a surgical stapler;
**characterized in that** the outer annular array (704; 1104) of staple forming pockets is arranged in a mirrored symmetry with the inner annular array (702; 1102) of staple forming pockets, such that:
the first innermost staple forming pocket (730; 1130) of the inner annular array (702; 1102) is angularly offset in a first circumferential direction from the second outermost staple forming pocket (710; 1110) of the inner annular array (702; 1102); and the second outermost staple forming pocket (750; 1150) of the outer annular array (704; 1104) is angularly offset in the same first circumferential direction from the first innermost staple forming pocket (770; 1170) of the outer annular array (704; 1104).

2. The apparatus of any preceding claim, wherein the first innermost staple forming pocket (730) of each pair of staple forming pockets of the inner annular array (702) overlaps with a corresponding first innermost staple forming pocket (770) of each pair of staple forming pockets of the outer annular array (704) along a radial dimension.

3. The apparatus of any preceding claim, wherein the second outermost staple forming pocket (710) of each pair of staple forming pockets of the inner annular array (702) overlaps with a corresponding second outermost staple forming pocket (750) of each pair of staple forming pockets of the outer annular array (704) along a radial dimension.

4. The apparatus of any preceding claim, wherein the first innermost staple forming pocket (730) of each pair of staple forming pockets of the inner annular array (702) overlaps with a corresponding second outermost staple forming pocket (710) of the same pair of staple forming pockets of the inner annular array (702) along a radial dimension.

5. The apparatus of any preceding claim, wherein the first innermost staple forming pocket (770) of each pair of staple forming pockets of the outer annular array (704) overlaps with a corresponding second outermost staple forming pocket (750) of the same pair of staple forming pockets of the outer annular array (704) along a radial dimension.

6. The apparatus of any preceding claim, wherein each staple forming pocket (710, 730, 750, 770) has a generally triangular shape defined in part by two generally flat sides that taper toward each other from a staple entry region toward a staple exit region.

7. The apparatus of any preceding claim, wherein each staple forming pocket (710, 730, 750, 770) is configured to deform a staple along three dimensions.

8. The apparatus of claim 7, wherein the three dimensions include a longitudinal dimension parallel to the longitudinal axis, a first orthogonal dimension extending orthogonally relative to the longitudinal axis, and a second orthogonal dimension extending orthogonally relative to the longitudinal axis.

9. The apparatus of any preceding claim, the first innermost staple forming pockets (1130) of the inner annular array (1102) are configured to deform a staple leg radially outwardly relative to the longitudinal axis, wherein the second outermost staple forming pockets (1110) of the inner annular array (1102) are configured to deform a staple leg radially inwardly relative to the longitudinal axis.

10. The apparatus of any preceding claim, wherein each pair of staple forming pockets in the inner annular array is substantially centered along a circumferential line (C_{L}) extending along the anvil surface at a constant radius from the longitudinal axis.

11. The apparatus of any preceding claim, wherein each first innermost staple forming pocket of the inner annular array is substantially centered along a circumferential line extending along the anvil surface at a constant radius from the longitudinal axis, wherein each second outermost staple forming pocket of the inner annular array is oriented obliquely relative to the same circumferential line extending along the anvil surface at a constant radius from the longitudinal axis.

12. The apparatus of any preceding claim, wherein each first innermost staple forming pocket (730) of the inner annular array (702) comprises a staple entry region (732) and a staple exit region (734), wherein the staple entry region (732) is wider than the staple exit region (734).

13. The apparatus of claim 12, wherein the staple entry region is defined in part by a first sidewall wall and an opposing second sidewall, wherein the staple exit region is defined in part by the first sidewall and an opposing third sidewall, wherein each first innermost staple forming pocket of the inner annular array further comprises a fourth sidewall providing an angled transition from the second sidewall to the third sidewall.

14. The apparatus of claim 1, further comprising a stapling head assembly (300), wherein the stapling head assembly (300) comprises:
(i) a deck surface (322), wherein the deck surface (322) is configured to cooperate with the anvil surface to compress tissue,
(ii) a plurality of staple openings (324) formed through the deck surface (322), wherein the staple openings (324) comprise:
(A) an inner annular array of staple openings corresponding to the inner annular array (702) of staple forming pockets, and
(B) an outer annular array of staple openings corresponding to the outer annular array (704) of staple forming pockets,
(iii) a plurality of staples, each staple being positioned in a corresponding staple opening (324) of the plurality of staple openings,
(iv) a staple driver (352) operable to drive the staples through the staple openings (324) and toward the anvil surface, and
(v) a shaft (330) configured to couple with the anvil shaft.

15. The apparatus of claim 1, wherein the inner annular array of staple forming pockets is configured such that the first innermost staple forming pocket of each pair of staple forming pockets does not overlap the second outermost staple forming pocket of the same pair of staple forming pockets along a radial dimension.

## Patentansprüche

1. Vorrichtung, umfassend:
(a) eine Ambossfläche, die dazu ausgelegt ist, Gewebe gegen eine Deckfläche zu drücken, wobei die Ambossfläche definiert:
(i) eine innere ringförmige Anordnung (702; 1102) aus Klammerformtaschen, wobei die innere ringförmige Anordnung aus Klammerformtaschen eine Vielzahl von Klammerformtaschenpaaren umfasst, wobei jedes Paar aus Klammerformtaschen in der inneren ringförmigen Anordnung umfasst:
(A) eine erste innerste Klammerformtasche (730; 1130), und
(B) eine zweite äußerste Klammerformtasche (710; 1110), und
(i) eine äußere ringförmige Anordnung (704; 1104) aus Klammerformtaschen, wobei die äußere ringförmige Anordnung aus Klammerformtaschen eine Vielzahl von Klammerformtaschenpaaren umfasst, wobei jedes Paar aus Klammerformtaschen in der äußeren ringförmigen Anordnung umfasst:
(A) eine erste innerste Klammerformtasche (770; 1170), und
(B) eine zweite äußerste Klammerformtasche (750; 1150);
(b) einen Ambossschaft, der sich entlang einer Längsachse erstreckt, wobei der Ambossschaft dazu ausgelegt ist, mit einer Klammerkopfanordnung eines chirurgischen Klammergeräts zu koppeln,
**dadurch gekennzeichnet, dass** die äußere ringförmige Anordnung (704; 1104) von Klammerformtaschen spiegelsymmetrisch zu der inneren ringförmigen Anordnung (702; 1102) aus Klammerformtaschen angeordnet ist, sodass:
die erste innerste Klammerformtasche (730; 1130) der inneren ringförmigen Anordnung (702; 1102) in einer ersten Umfangsrichtung von der zweiten äußersten Klammerformtasche (710; 1110) der inneren ringförmigen Anordnung (702; 1102) winklig versetzt ist; und die zweite äußerste Klammerformtasche (750; 1150) der äußeren ringförmigen Anordnung (704; 1104) in derselben ersten Umfangsrichtung von der ersten innersten Klammerformtasche (770; 1170) der äußeren ringförmigen Anordnung (704; 1104) winklig versetzt ist.

2. Vorrichtung nach einem vorhergehenden Anspruch, wobei die erste innerste Klammerformtasche (730) eines jeden Paares aus Klammerformtaschen der inneren ringförmigen Anordnung (702) mit einer entsprechenden ersten innersten Klammerformtasche (770) eines jeden Paares aus Klammerformtaschen der äußeren ringförmigen Anordnung (704) entlang einer radialen Abmessung überlappt.

3. Vorrichtung nach einem vorhergehenden Anspruch, wobei die zweite äußerste Klammerformtasche (710) eines jeden Paares aus Klammerformtaschen der inneren ringförmigen Anordnung (702) mit einer entsprechenden zweiten äußersten Klammerformtasche (750) eines jeden Paares aus Klammerformtaschen der äußeren ringförmigen Anordnung (704) entlang einer radialen Abmessung überlappt.

4. Vorrichtung nach einem vorhergehenden Anspruch, wobei die erste innerste Klammerformtasche (730) eines jeden Paares aus Klammerformtaschen der inneren ringförmigen Anordnung (702) mit einer entsprechenden zweiten äußersten Klammerformtasche (710) desselben Paares aus Klammerformtaschen der inneren ringförmigen Anordnung (702) entlang einer radialen Abmessung überlappt.

5. Vorrichtung nach einem vorhergehenden Anspruch, wobei die erste innerste Klammerformtasche (770) eines jeden Paares aus Klammerformtaschen der äußeren ringförmigen Anordnung (704) mit einer entsprechenden zweiten äußersten Klammerformtasche (750) desselben Paares aus Klammerformtaschen der äußeren ringförmigen Anordnung (704) entlang einer radialen Abmessung überlappt.

6. Vorrichtung nach einem vorhergehenden Anspruch, wobei jede Klammerformtasche (710, 730, 750, 770) eine im Allgemeinen dreieckige Form hat, die teilweise von zwei im Allgemeinen ebenen Seiten definiert wird, die von einem Klammereintrittsbereich in Richtung eines Klammeraustrittsbereichs konisch aufeinanderzulaufen.

7. Vorrichtung nach einem vorhergehenden Anspruch, wobei jede Klammerformtasche (710, 730, 750, 770) dazu ausgelegt ist, eine Klammer entlang von drei Abmessungen zu verformen.

8. Vorrichtung nach Anspruch 7, wobei die drei Abmessungen eine Längsabmessung parallel zur Längsachse, eine erste orthogonale Abmessung, die sich orthogonal bezogen auf die Längsachse erstreckt, und eine zweite orthogonale Abmessung, die sich orthogonal bezogen auf die Längsachse erstreckt, aufweisen.

9. Vorrichtung nach einem vorhergehenden Anspruch, wobei die ersten innersten Klammerformtaschen (1130) der inneren ringförmigen Anordnung (1102) dazu ausgelegt sind, einen Klammerschenkel bezogen auf die Längsachse radial nach außen zu verformen, wobei die zweiten äußersten Klammerformtaschen (1110) der inneren ringförmigen Anordnung (1102) dazu ausgelegt sind, einen Klammerschenkel bezogen auf die Längsachse radial nach innen zu verformen.

10. Vorrichtung nach einem vorhergehenden Anspruch, wobei jedes Paar aus Klammerformtaschen in der inneren ringförmigen Anordnung im Wesentlichen entlang einer Umfangslinie (C_{L}) zentriert ist, die sich entlang der Ambossfläche in einem konstanten Radius von der Längsachse erstreckt.

11. Vorrichtung nach einem vorhergehenden Anspruch, wobei jede erste innere Klammerformtasche der inneren ringförmigen Anordnung im Wesentlichen entlang einer Umfangslinie zentriert ist, die sich entlang der Ambossfläche in einem konstanten Radius von der Längsachse erstreckt, wobei jede zweite äußere Klammerformtasche der inneren ringförmigen Anordnung schräg bezogen auf dieselbe Umfangslinie ausgerichtet ist, die sich entlang der Ambossfläche in einem konstanten Radius von der Längsachse erstreckt.

12. Vorrichtung nach einem vorhergehenden Anspruch, wobei jede erste innerste Klammerformtasche (730) der inneren ringförmigen Anordnung (702) einen Klammereintrittsbereich (732) und einen Klammeraustrittsbereich (734) umfasst, wobei der Klammereintrittsbereich (732) breiter ist als der Klammeraustrittsbereich (734).

13. Vorrichtung nach Anspruch 12, wobei der Klammereintrittsbereich teilweise von einer ersten Seitenwand und einer gegenüberliegenden zweiten Seitenwand definiert wird, wobei der Klammeraustrittsbereich teilweise von der ersten Seitenwand und einer gegenüberliegenden dritten Seitenwand definiert wird, wobei jede erste innerste Klammerformtasche der inneren ringförmigen Anordnung ferner eine vierte Seitenwand umfasst, die einen winkligen Übergang von der zweiten Seitenwand zu der dritten Seitenwand bereitstellt.

14. Vorrichtung nach Anspruch 1, ferner umfassend eine Klammerkopfanordnung (300), wobei die Klammerkopfanordnung (300) umfasst:
(i) eine Deckfläche (322), wobei die Deckfläche (322) dazu ausgelegt ist, mit der Ambossfläche zusammenzuwirken, um Gewebe zu komprimieren,
(ii) eine Vielzahl von Klammeröffnungen (324), die durch die Deckfläche (322) gebildet sind, wobei die Klammeröffnungen (324) umfassen:
(A) eine innere ringförmige Anordnung von Klammeröffnungen, die der inneren ringförmigen Anordnung (702) von Klammerformtaschen entspricht, und
(B) eine äußere ringförmige Anordnung von Klammeröffnungen, die der äußeren ringförmigen Anordnung (704) von Klammerformtaschen entspricht,
(iii) eine Vielzahl von Klammern, wobei jede Klammer in einer entsprechenden Klammeröffnung (324) der Vielzahl von Klammeröffnungen positioniert ist,
(iv) einen Klammertreiber (352), der bedienbar ist, um die Klammern durch die Klammeröffnungen (324) und in Richtung der Ambossfläche zu treiben, und
(v) einen Schaft (330), der dazu ausgelegt ist, mit dem Ambossschaft zu koppeln.

15. Vorrichtung nach Anspruch 1, wobei die innere ringförmige Anordnung von Klammerformtaschen derart ausgelegt ist, dass die erste innerste Klammerformtasche eines jeden Paares von Klammerformtaschen mit der zweiten äußersten Klammerformtasche desselben Paares von Klammerformtaschen entlang einer radialen Abmessung nicht überlappt.

## Revendications

1. Appareil comprenant :
(a) une surface d'enclume conçue pour comprimer un tissu contre une surface de pont, dans lequel la surface d'enclume définit :
(i) un réseau annulaire interne (702 ; 1102) de poches de formation d'agrafes, dans lequel le réseau annulaire interne de poches de formation d'agrafes comprend une pluralité de paires de poches de formation d'agrafes, dans lequel chaque paire de poches de formation d'agrafes dans le réseau annulaire interne comprend :
(A) une première poche de formation d'agrafe la plus interne (730 ; 1130), et
(B) une seconde poche de formation d'agrafe la plus externe (710 ; 1110), et
(ii) un réseau annulaire externe (704 ; 1104) de poches de formation d'agrafes, dans lequel le réseau annulaire externe de poches de formation d'agrafes comprend une pluralité de paires de poches de formation d'agrafes, dans lequel chaque paire de poches de formation d'agrafes dans le réseau annulaire externe comprend :
(A) une première poche de formation d'agrafe la plus interne (770 ; 1170), et
(B) une seconde poche de formation d'agrafe la plus externe (750 ; 1150) ; et
(b) un arbre d'enclume s'étendant le long d'un axe longitudinal, dans lequel l'arbre d'enclume est conçu pour s'accoupler avec un ensemble tête d'agrafage d'une agrafeuse chirurgicale ;
**caractérisé en ce que** le réseau annulaire externe (704 ; 1104) de poches de formation d'agrafes est agencé dans une symétrie en miroir avec le réseau annulaire interne (702 ; 1102) de poches de formation d'agrafes, telle sorte que :
la première poche de formation d'agrafe la plus interne (730 ; 1130) du réseau annulaire interne (702 ; 1102) est décalée angulairement dans une première direction circonférentielle par rapport à la seconde poche de formation d'agrafe la plus externe (710 ; 1110) du réseau annulaire interne (702 ; 1102) ; et la seconde poche de formation d'agrafe la plus externe (750 ; 1150) du réseau annulaire externe (704 ; 1104) est décalée angulairement dans la même première direction circonférentielle par rapport à la première poche de formation d'agrafe la plus interne (770 ; 1170) du réseau annulaire externe (704 ; 1104).

2. Appareil selon une quelconque revendication précédente, dans lequel la première poche de formation d'agrafe la plus interne (730) de chaque paire de poches de formation d'agrafes du réseau annulaire interne (702) chevauche une première poche de formation d'agrafe la plus interne correspondante (770) de chaque paire de poches de formation d'agrafes du réseau annulaire externe (704) le long d'une dimension radiale.

3. Appareil selon une quelconque revendication précédente, dans lequel la seconde poche de formation d'agrafe la plus externe (710) de chaque paire de poches de formation d'agrafes du réseau annulaire interne (702) chevauche une seconde poche de formation d'agrafe la plus externe correspondante (750) de chaque paire de poches de formation d'agrafes du réseau annulaire externe (704) le long d'une dimension radiale.

4. Appareil selon une quelconque revendication précédente, dans lequel la première poche de formation d'agrafe la plus interne (730) de chaque paire de poches de formation d'agrafes du réseau annulaire interne (702) chevauche une seconde poche de formation d'agrafe la plus externe correspondante (710) de la même paire de poches de formation d'agrafes du réseau annulaire interne (702) le long d'une dimension radiale.

5. Appareil selon une quelconque revendication précédente, dans lequel la première poche de formation d'agrafe la plus interne (770) de chaque paire de poches de formation d'agrafes du réseau annulaire externe (704) chevauche une seconde poche de formation d'agrafe la plus externe correspondante (750) de la même paire de poches de formation d'agrafes du réseau annulaire externe (704) le long d'une dimension radiale.

6. Appareil selon une quelconque revendication précédente, dans lequel chaque poche de formation d'agrafe (710, 730, 750, 770) a une forme généralement triangulaire définie en partie par deux côtés généralement plats qui s'effilent l'un vers l'autre à partir d'une région d'entrée d'agrafes vers une région de sortie d'agrafes.

7. Appareil selon une quelconque revendication précédente, dans lequel chaque poche de formation d'agrafe (710, 730, 750, 770) est conçue pour déformer une agrafe le long de trois dimensions.

8. Appareil selon la revendication 7, dans lequel les trois dimensions comprennent une dimension longitudinale parallèle à l'axe longitudinal, une première dimension orthogonale s'étendant orthogonalement par rapport à l'axe longitudinal, et une seconde dimension orthogonale s'étendant orthogonalement par rapport à l'axe longitudinal.

9. Appareil selon une quelconque revendication précédente, les premières poches de formation d'agrafes les plus internes (1130) du réseau annulaire interne (1102) sont conçues pour déformer une patte d'agrafe radialement vers l'extérieur par rapport à l'axe longitudinal, dans lequel les secondes poches de formation d'agrafes les plus externes (1110) du réseau annulaire interne (1102) sont conçues pour déformer une patte d'agrafe radialement vers l'intérieur par rapport à l'axe longitudinal.

10. Appareil selon une quelconque revendication précédente, dans lequel chaque paire de poches de formation d'agrafes dans le réseau annulaire interne est sensiblement centrée le long d'une ligne circonférentielle (C_{L}) s'étendant le long de la surface de l'enclume à un rayon constant à partir de l'axe longitudinal.

11. Appareil selon une quelconque revendication précédente, dans lequel chaque première poche de formation d'agrafe la plus interne du réseau annulaire interne est sensiblement centrée le long d'une ligne circonférentielle s'étendant le long de la surface de l'enclume à un rayon constant à partir de l'axe longitudinal, dans lequel chaque seconde poche de formation d'agrafe la plus externe du réseau annulaire interne est orientée obliquement par rapport à la même ligne circonférentielle s'étendant le long de la surface de l'enclume à un rayon constant à partir de l'axe longitudinal.

12. Appareil selon une quelconque revendication précédente, dans lequel chaque première poche de formation d'agrafe la plus interne (730) du réseau annulaire interne (702) comprend une région d'entrée d'agrafes (732) et une région de sortie d'agrafes (734), dans lequel la région d'entrée d'agrafes (732) est plus large que la région de sortie d'agrafes (704).

13. Appareil selon la revendication 12, dans lequel la région d'entrée d'agrafes est définie en partie par une première paroi latérale et une deuxième paroi latérale opposée, dans lequel la région de sortie d'agrafes est définie en partie par la première paroi latérale et une troisième paroi latérale opposée, dans lequel chaque première poche de formation d'agrafe la plus interne du réseau annulaire interne comprend en outre une quatrième paroi latérale fournissant une transition angulaire de la deuxième paroi latérale à la troisième paroi latérale.

14. Appareil selon la revendication 1, comprenant en outre un ensemble tête d'agrafage (300), dans lequel l'ensemble tête d'agrafage (300) comprend :
(i) une surface de pont (322), dans lequel la surface de pont (322) est conçue pour coopérer avec la surface de l'enclume afin de comprimer un tissu,
(ii) une pluralité d'ouvertures d'agrafes (324) formées à travers la surface de pont (322), dans lequel les ouvertures d'agrafes (324) comprennent :
(A) un réseau annulaire interne d'ouvertures d'agrafes correspondant au réseau annulaire interne (702) de poches de formation d'agrafes, et
(B un réseau annulaire externe d'ouvertures d'agrafes correspondant au réseau annulaire externe (704) de poches de formation d'agrafes,
(iii) une pluralité d'agrafes, chaque agrafe étant positionnée dans une ouverture d'agrafe correspondante (324) de la pluralité d'ouvertures d'agrafes,
(iv) un organe d'entraînement d'agrafes (352) pouvant entraîner les agrafes à travers les ouvertures d'agrafes (324) et vers la surface de l'enclume, et
(v) un arbre (330) conçu pour s'accoupler avec l'arbre de l'enclume.

15. Appareil selon la revendication 1, dans lequel le réseau annulaire interne de poches de formation d'agrafes est conçu de telle sorte que la première poche de formation d'agrafe la plus interne de chaque paire de poches de formation d'agrafes ne chevauche pas la seconde poche de formation d'agrafe la plus externe de la même paire de poches de formation d'agrafes le long d'une dimension radiale.
